Europäisches Patentamt

European Patent Office

Office européen des brevets

(19)

(11) **EP 0 354 109 B1**

(12) **FASCICULE DE BREVET EUROPEEN**

(45) Date de publication et mention
de la délivrance du brevet:
**17.04.1996 Bulletin 1996/16**

(51) Int Cl.6: **C12N 15/62**, C12N 15/48,
C12N 15/36, A61K 39/00,
G01N 33/00

(21) Numéro de dépôt: **89402171.6**

(22) Date de dépôt: **28.07.1989**

(54) **Particules HBsAg recombinantes hybrides ayant des caractéristiques morphologiques de l'antigène HBsAg contenant une séquence immunogène induisant des anticorps neutralisants dirigés contre HIV ou susceptible d'être reconnue par de tels anticorps. Séquences nucléotidiques codant pour de telles particules. Vaccins les contenant**

Rekombinante HBsAg Hybridpartikel, die morphologische Eigenschaften von HBsAg-Antigen haben und eine immunogene Sequenz enthalten, die neutralisierende Antikörper gegen HIV induziert oder die von diesen Antikörpern erkannt ist, Nukleotide Sequenzen, die diese Partikel codieren und sie enthaltende Impfstoffe

Recombinant HBsAg hybrid particles having morphological characteristics of the HBsAg antigen and containing an immunogenic sequence which induces neutralizing antibodies directed against HIV or susceptible of being recognized by such antibodies. Nucleotide sequences coding for such particles and vaccines containing them

(84) Etats contractants désignés:
**AT BE CH DE ES FR GB GR IT LI LU NL SE**

(30) Priorité: **29.07.1988 FR 8810336**

(43) Date de publication de la demande:
**07.02.1990 Bulletin 1990/06**

(73) Titulaires:
- **INSTITUT PASTEUR**
  **F-75724 Paris Cédex 15 (FR)**
- **INSTITUT NATIONAL DE LA SANTE ET DE LA RECHERCHE MEDICALE (INSERM)**
  **F-75654 Paris Cédex 13 (FR)**

(72) Inventeurs:
- **Tiollais, Pierre**
  **F-75013 Paris (FR)**
- **Michel, Marie-Louise**
  **F-75018 Paris (FR)**
- **Mancini, Maryline**
  **F-75012 Paris (FR)**
- **Sobczak, Eliane**
  **F-75011 Paris (FR)**

(74) Mandataire: **Gutmann, Ernest et al**
**Ernest Gutmann - Yves Plasseraud S.A.**
**3, rue Chauveau-Lagarde**
**F-75008 Paris (FR)**

(56) Documents cités:
| EP-A- 0 038 765 | EP-A- 0 175 261 |
| EP-A- 0 181 150 | EP-A- 0 199 301 |
| EP-A- 0 201 416 | EP-A- 0 269 520 |
| EP-A- 0 278 940 | WO-A-88/05440 |

- **PROCEEDINGS OF THE NATL. ACADEMY OF SCIENCES USA, vol. 85, novembre 1988, Washington, DC (US); M.L. MICHEL et al., pp. 7957-7961**
- **PROCEEDINGS OF THE NATL. ACADEMY OF SCIENCES USA, vol. 84, juin 1987, Washington, DC (US); K.B. CEASE et al., pp. 4249-4253**

## Description

La présente invention concerne des particules recombinantes hybrides ayant les caractéristiques morphologiques de l'antigène HBsAg comprenant des séquences d'acides aminés immunogènes induisant des anticorps neutralisants dirigés contre les rétrovirus de la famille de HIV ou capables d'être reconnues par de tels anticorps.

L'invention vise également des acides nucléiques recombinants ou vecteurs recombinants, comprenant eux-mêmes les insérats formés par des acides nucléiques recombinants hybrides, éventuellement sous le contrôle d'un promoteur reconnu par les polymérases d'un hôte cellulaire eucaryote, ces acides nucléiques recombinants portant l'information génétique requise pour rendre cet hôte cellulaire eucaryote apte à produire les particules hybrides recombinantes sus-mentionnées. L'invention a aussi pour objet des hôtes cellulaires eucaryotes transformés par les vecteurs recombinants précédents.

La présente invention propose, par l'intermédiaire des particules recombinantes ci-dessus, de nouveaux moyens de prévention contre le SIDA ou contre les symptômes précurseurs du SIDA notamment des syndrômes lymphadénopathiques (SLA).

L'invention concerne plus particulièrement des particules recombinantes de ce type qui ont la capacité d'induire la formation d'anticorps neutralisants vis-à-vis des rétrovirus HIV et leur application pour l'élaboration de compositions vaccinantes pour lutter contre une infection due à un rétrovirus HIV.

L'invention a également pour objet des peptides et polypeptides adéquats ayant des propriétés immunogènes protectrices contre le SIDA, notamment des polypeptides de l'enveloppe d'un rétrovirus HIV ou SIV pour la réalisation des particules recombinantes ci-dessus.

L'invention se rapporte également à des acides nucléiques codant pour ces peptides et polypeptides ainsi qu'aux vecteurs les contenant.

L'invention vise également l'utilisation des moyens ci-dessus pour réaliser le diagnostic in vitro d'une infection due à HIV.

On sait maintenant que des agents étiologiques responsables du développement de syndrômes de lymphadénopathies (SLA) précèdent souvent le développement de syndrôme d'immunodéficience acquise (SIDA).

On a désigné par HIV (abréviation anglaise de "Human Immunodéficiency Virus") plusieurs rétrovirus humains susceptibles, dans certaines conditions, de provoquer un SLA, éventuellement relayé par un syndrôme d'immunodéficience acquise (SIDA) chez l'homme.

Ainsi un premier virus dénommé LAV-1 ou HIV-1 a été isolé et décrit dans la demande de brevet GB.83/24.800 et une demande EP.84/401.834 du 14/09/84. Ce virus a également été décrit par F.Barre Sinoussi et al. dans Science, 220 n° 45-99, 20, pages 868-871.

Des variants de ce virus HIV-1 désignés par LAV ELI et LAV MAL, ont également été isolés, caractérisés et décrits dans la demande de brevet EP.84/401.834.

L'isolement et la caractérisation de rétrovirus appartenant à une classe distincte et n'ayant qu'une parenté immunologique réduite avec les précédents, ont été décrits dans la demande de brevet européen n° 87/400.151.4. publiée sous le n° 239.425. Ces rétrovirus qui ont été regroupés sous la désignation HIV-2, ont été isolés chez plusieurs malades africains présentant des symptômes d'une lymphadénopathie ou d'un SIDA.

L'étude de ces rétrovirus HIV, l'isolement et l'analyse de leurs séquences nucléotidiques, ainsi que la caractérisation de leurs protéines antigéniques a permis d'effectuer des études comparatives entre les différentes souches obtenues au niveau de leur parenté ou au contraire de leurs spécificités tant structurales que fonctionnelles.

Ces rétrovirus HIV-1 et HIV-2 ont également été étudiés en comparaison avec un rétrovirus d'origine simienne (désigné par l'abréviation SIV ou STLV-III) et cette étude a permis de montrer une parenté immunologique entre les protéines et glycoprotéines du rétrovirus HIV-2 et de ses variants et celles du rétrovirus SIV. La glycoprotéine d'enveloppe du rétrovirus SIV réagit immunologiquement avec des anticorps dirigés contre la glycoprotéine d'enveloppe du rétrovirus HIV-2 et vice-versa. En revanche les glycoprotéines d'enveloppe du rétrovirus SIV ou du rétrovirus HIV-2 ne donnent pas lieu à des réactions immunologiques croisées avec les glycoprotéines d'enveloppe du rétrovirus HIV-1.

Les études réalisées sur la structure des antigènes de surface de ces rétrovirus ont conduit les inventeurs à s'intéresser plus spécialement aux propriétés immunogènes de différents peptides et polypeptides appartenant à ces antigènes, notamment aux glycoprotéines d'enveloppe, ou aux glycoprotéines transmembranaires de ces rétrovirus.

Différents peptides et polypeptides caractéristiques dont les séquences sont contenues dans les structures peptidiques de ces glycoprotéines, soit directement issus de ces rétrovirus, soit synthétisés par voie chimique, présentent une immunogénicité relativement faible.

Dans le cadre de la présente invention les inventeurs ont recherché et défini des moyens susceptibles de renforcer l'immunogénicité de tels peptides ou polypeptides dérivés des glycoprotéines d'enveloppe ou des glycoprotéines membranaires de HIVs - ou élaborés à partir de la connaissance de leurs séquences respectives,- ou encore de séquences fonctionnellement voisines, en particulier pour les rendre aptes à induire in vivo la formation d'anticorps neutralisants vis-à-vis d'un ou plusieurs rétrovirus du type HIV.

Plus précisément, les inventeurs ont orienté leurs travaux vers la mise au point d'une structure biologique mixte dont l'un des éléments est constitué par les susdits peptides ou polypeptides, l'autre par des particules ayant les propriétés immunogéniques et immunologiques essentielles de l'antigène de surface (souvent désigné par l'abréviation HBsAg ou encore plus simplement HBs) du virus de l'hépatite B.

Des particules ayant les propriétés morphologiques et immunogènes essentielles de l'antigène HBsAg naturel, et produites par expression du gène S du virus de l'hépatite B dans des cellules eucaryotes, ont été décrites dans le brevet européen n° 0038765 déposé le 22 avril 1981. On rappelle brièvement que ces particules HBs ont une taille approximative de 17 à 25, notamment 22 nm de diamètre. Elles ont également été décrites dans l'article scientifique correspondant de M.F. DUBOIS et al, (1980), Proc. Natl. Acad. Sci., USA, 77, 4549-4553). Des particules semblables ont été obtenues dans des levures (P. VALENZUELA et al, (1982), Nature, 298, 347-350) ou par l'intermédiaire de virus recombinants (G.L. SMITH et al, (1983), Nature, 302, 490-495).

Une méthode pour produire ces particules comprend la transformation de cellules eucaryotes par un vecteur approprié, contenant le gène S sous la dépendance d'un promoteur efficace dans ces cellules eucaryotes, la culture des cellules transformées et la récupération des particules produites, soit à partir des cellules préalablement lysées, soit à partir du milieu de culture, lorsque les particules y ont été excrétées par les lignées cellulaires utilisées (notamment dans le cas de l'utilisation de lignées cellulaires de singe, de hamster, de souris etc...)

Le polypeptide majeur des particules HBs, codé par le gène S a un poids moléculaire (PM) d'environ 25400 daltons. Ce poids moléculaire est de l'ordre de 27000 daltons lorsque la protéine majeure est sous forme glycosylée.

Dans le cadre de l'invention, les inventeurs se sont également intéressés à la préparation de protéines de PM plus élevé (de l'ordre de 34000 daltons), contenant non seulement la séquence polypeptidique du susdit polypeptide majeur codée pour la région S, ayant la même extrémité C-terminale que le polypeptide majeur et en outre une séquence supplémentaire de 55 acides aminés en position N-terminale (STIBBE X. et GERLICH W.H., (1983), J. Virology, 46, 626-628) codée par la région pré-S2 du génome de l'hépatite B. Les inventeurs ont aussi étudié des particules résultant de la transcription des régions S, pré-S1 et pré-S2 (l'ensemble de ces deux domaines est appelé région pré-S).

Les protéines recombinantes ci-dessus s'assemblent en particules d'une taille approximative de 22 nm.

L'invention résulte de la conjonction dans une entité commune de facteurs concourant au résultat recherché à savoir :

1/ un support consistant en des particules de HBsAg comprenant plusieurs sites épitopiques T et B, dont l'immunogénicité protectrice est acquise, quand elles n'ont pas été modifiées (et lorsque ses protéines de surface comprennent la séquence d'acides aminés codée par la région pré-S du génome du virus de l'hépatite B),

2/ un épitope T immunogène dérivé d'une glycoprotéine d'enveloppe de HIV,

3/ un épitope B appartenant également à une glycoprotéine d'enveloppe de HIV et selon la forme de réalisation de l'invention,

4/ avantageusement d'un site d'attachement au récepteur CD4 de lymphocytes T4. Ce site, en combinaison avec les facteurs précédents, participerait à l'induction in vivo des anticorps neutralisants contre HIV et à l'induction d'une réponse cellulaire, accompagnée d'une action bloquante des anticorps formés, dès lors qu'ils sont dirigés contre un site de fixation du virus.

Le but que les inventeurs se sont fixé a été atteint grâce à la réalisation de particules hybrides recombinantes présentant les caractéristiques morphologiques essentielles des particules de HBsAg, et comprenant, affleurant à la surface de ces particules, l'essentiel de la séquence d'acides aminés (S) codée par la région S et le cas échéant, tout ou partie de la séquence d'acides aminés codée par la région pré-S du génome du virus de l'hépatite B en amont de ladite séquence d'acides aminés (S), ces particules recombinantes étant caractérisées en ce qu'elles comprennent en outre, une séquence d'acides aminés immunogène induisant des anticorps neutralisants vis-à-vis d'un rétrovirus de la famille HIV, ou susceptible d'être reconnue par de tels anticorps, cette séquence comportant elle-même au moins un épitope susceptible d'être reconnu par les lymphocytes B, au moins un épitope susceptible d'être reconnu par les lymphocytes T, cette séquence immunogène étant, soit insérée dans la séquence d'acides aminés pré-S, soit substituée tout ou partie de la protéine pré-S.

De façon particulièrement avantageuse les particules hybrides recombinantes ci-dessus comprennent en outre un site d'attachement au récepteur CD4 de lymphocytes T4 humains.

L'invention concerne aussi un vecteur recombinant pour la transformation ultérieure de cellules compétentes, alors aptes à produire de telles particules.

Le vecteur recombinant selon l'invention comprend un fragment d'acide nucléique correspondant à la région S et le cas échéant à tout ou partie de la région pré-S de l'antigène majeur de surface du virus de l'hépatite B, et permettant lorsqu'il est introduit dans un hôte cellulaire eucaryote compétent, d'induire la production de particules, ayant les caractéristiques morphologiques essentielles de l'antigène HBsAg et comprenant une séquence d'acides aminés caractéristique de la protéine S de l'antigène HBsAg, à l'occasion de la culture de l'hôte cellulaire ainsi transformé et, de préférence, l'excrétion de ces particules dans le milieu de culture, ce vecteur recombinant étant caractérisé :

- en ce qu'il comprend en outre une séquence nucléotidique exogène codant pour une séquence d'acides aminés immunogène induisant des anticorps neutralisants vis-à-vis d'un rétrovirus de la famille de HIV ou susceptible d'être reconnue par de tels anticorps, comprenant au moins un épitope susceptible d'être reconnu par les lymphocytes B, et au moins un épitope susceptible d'être reconnu par les lymphocytes T, et
- en ce que ladite séquence nucléotidique exogène est soit insérée dans ladite région pré-S, soit substituée à tout ou partie de la dite région pré-S.

Avantageusement, la séquence nucléotidique exgène code pour une séquence d'acides aminés immunogène telle que définie ci-dessus et qui comprend en outre un site d'attachement au récepteur CD4.

Dans un mode de réalisation préféré du vecteur recombinant, le fragment d'acide nucléique correspondant à la région S et le cas échéant à tout ou partie de la région pré-S est placé sous le contrôle d'un promoteur reconnu par les polymérases d'un hôte cellulaire eucaryote.

L'incorporation des séquences caractéristiques de HIV dans la région pré-S (ou la substitution partielle ou totale de cette région pré-S par lesdites séquences caractéristiques) permet à la fois l'expression et l'exposition des séquences issue de HIV à la surface des particules ayant conservé les caractéristiques essentielles des particules HBsAg, et ce dans une conformation autorisant la conservation de leur immunogénicité naturelle et leur amplification, au point de les rendre aptes à induire in vivo la formation d'anticorps neutralisants vis-à-vis d'un rétrovirus HIV.

Avantageusement on a recours à des vecteurs recombinants comprenant des séquences nucléotidiques exogènes issues de HIV, choisies parmi celles qui présentent un grand degré de conservation au sein de différents isolats de rétrovirus HIV.

La longueur des séquences nucléotidiques insérées dans l'enchaînement nucléotidique comprenant la région S et le cas échéant tout ou partie de la région pré-S, doit être compatible avec le maintien des propriétés structurales et conformationnelles (morphologiques) essentielles des particules porteuses HBs. Cette longueur dépend dans une certaine mesure de la partie délétée de la région pré-S à titre indicatif ... mais non limitatif ... cette longueur correspond à une séquence de 5 à 100 résidus aminoacides. La longueur de la séquence insérée peut dans certaines formes de réalisation de l'invention, excéder celle de la séquence délétée.

Dans un mode de réalisation préféré des vecteurs recombinants selon l'invention, la séquence nucléotidique exogène est insérée dans la phase de lecture appropriée, dans la région pré-S (ou en amont du gène S) sous le contrôle d'un promoteur issu du SV40.

Dans un mode de réalisation particulier de l'invention, l'acide nucléique recombinant est caractérisé en ce que la séquence nucléotidique exogène (issue de HIV) est substituée à la plus grande partie, sinon la totalité de la région pré-S2.

En d'autres termes, la région pré-S2 subsistant après l'insertion de ladite séquence nucléotidique exogène est pratiquement entièrement délétée, la séquence nucléotidique exogène étant alors directement intercalée entre la région S, et la région pré-S1. Par séquence nucléotidique exogène, on entend les séquences dont les définitions sont données dans les pages précédentes ainsi que celles qui seront exemplifiées dans la suite. Entrent toutefois dans le cadre de l'invention des vecteurs transformés susceptibles de favoriser l'intégration dans le fragment d'acide nucléiquede HBs, des susdites séquences nucléotides exogènes.

Des vecteurs recombinants particulièrement adaptés à l'expression des séquences nucléotidiques exogènes dans le cadre des propriétés recherchées, résultent de la recombinaison d'un vecteur d'expression connu de HBsAg, désigné par pSVS (voir à ce sujet la référence 11) et d'une séquence exogène, issu de HIV-2 portée par un vecteur de clonage M13tg130.

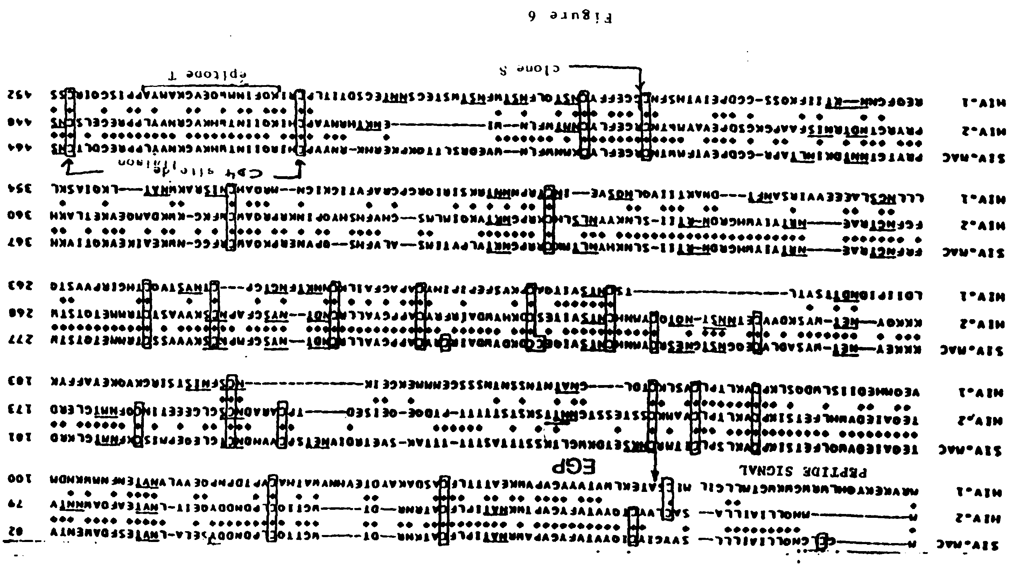

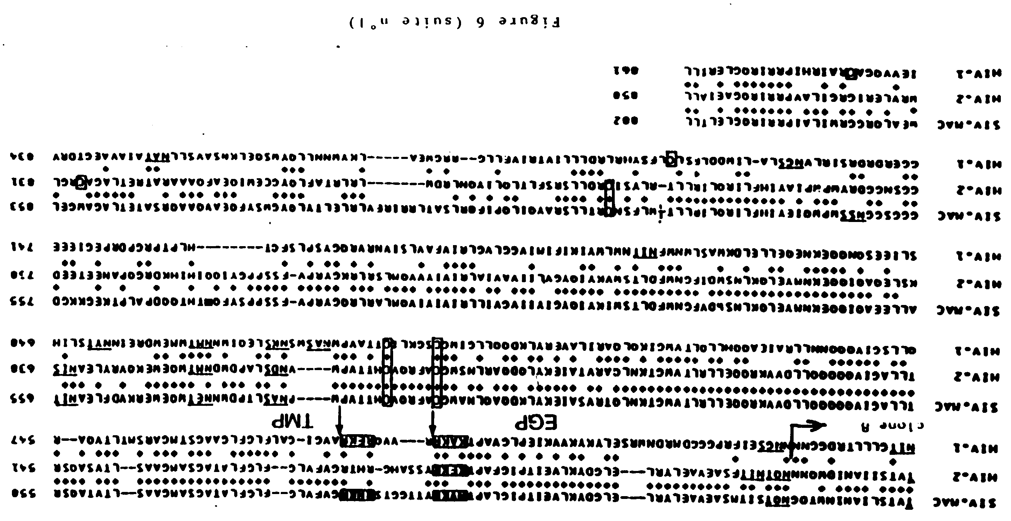

Un tel vecteur résulte de l'insertion de la séquence exogène dans la région pré-S2 de HBsAg (vecteur pM2S qui contient la séquence pré-S1) ou de la substitution d'une partie de la région pré-S par la séquence exogène (pSV2S dans lequel la région pré-S1 a été délétée). Ces deux vecteurs, non limitatifs des possibilités de réalisation de l'invention, contiennent une séquence nucléotidique exogène caractéristique de la glycoprotéine externe d'enveloppe de HIV-1 obtenue par clonage et désignée par "clone 8". Elle est présentée à la figure 6.

D'autres constructions peuvent être obtenues en remplaçant la séquence de HIV-1 ci-dessus par les séquences nucléotidiques de HIV-2 ou de SIV correspondantes, ou encore par des séquences modifiées, immunologiquement équivalentes, capables cependant in vivo la production d'anticorps neutralisants dirigés contre un rétrovirus HIV.

A titre d'exemple, les séquences insérées de façon préférée dans les enchaînements de nucléotides ci-dessus sont les séquences nucléotidiques codant pour l'une des séquences d'acides aminés suivantes :

$$X_1 \quad \text{CHIRQIINTWHKVGKNVYLPPREGDLTC} \quad Z_1$$
$$X_2 \quad \text{CHIKQIINTWHKVGRNVYLPPREGELSC} \quad Z_2$$
$$X_3 \quad \text{CRIKQFINMWQEVGKAMYAPPISGQIRC} \quad Z_3$$

dans lesquelles les couples $X_1$, $Z_1$ ; $X_2$, $Z_2$ et $X_3$, $Z_3$ représentent soit des groupements COOR ou NH$_2$, soit des séquences d'acides aminés normalement respectivement associées aux parties centrales correspondantes des séquences sus-indiquées dans les glycoprotéines des rétrovirus SIV, HIV-2, HIV-1 qui normalement les contiennent : ces séquences comprenant notamment au total, jusqu'à 100 acides aminés.

D'autres séquences nucléotidiques également préférées sont celles qui codent pour les séquences d'acides aminés suivantes :

RGEFLYCKMNWFLNWVEDRSLTTQKPKERHKRNYVPCHIRQIINTWHKVGKNVYLP PREGDLTCNSTVTSLIANINWTDG ;

RGEFLYCNMTWFLNWIENKTHRNYAPCHIKQIINTWHKVGRNVYLPPREGELSCNS TVTSIIANIDWQNN ;

GGEFFYCNSTQLFNSTWFNSTWSTEGSNNTEGSDTITLPCRIKQFINMWQEVGKAM YAPPISGQIRCSSNITGLLLTRDGGNN ;

Dans un mode de réalisation particulier des particules ci-dessus la séquence d'acides aminés exogène est soit insérée dans la séquence peptidique codée par la région pré-S2 du génome du virus de l'hépatite B, soit substituée à tout ou partie de cette séquence peptidique.

De façon préférée, les particules hybrides recombinantes contiennent une séquence d'acides aminés exogène choisie parmi celles qui ont déjà été indiquées à titre d'exemples préférés :

$$X_1 \quad \text{CHIRQIINTWHKVGKNVYLPPREGDLTC} \quad Z_1$$
$$X_2 \quad \text{CHIKQIINTWHKVGRNVYLPPREGELSC} \quad Z_2$$
$$X_3 \quad \text{CRIKQFINMWQEVGKAMYAPPISGQIRC} \quad Z_3$$

Ces séquences particulières présentent des caractéristiques fonctionnelles intéressantes, notamment en ce qu'elles contiennent dans une séquence commune issue de HIV un site d'attachement aux lymphocytes T et récepteurs CD4 et un épitope T très proche l'une de l'autre.

Une extension de l'application de l'invention consiste dans la réalisation d'un vecteur recombinant exogène caractéristique d'une glycoprotéine d'enveloppe de HIV mais dépourvu de la séquence codant pour le site d'attachement au récepteur CD4. Une telle séquence exogène insérée dans la région pré-S définie ci-dessus peut conduire de façon intéressante à la formation de particules hybrides recombinantes capables de présenter à leur surface les épitopes susceptibles d'être reconnus par les lymphocytes B et les lymphocytes T et capables respectivement de contribuer à l'induction d'anticorps ou d'une réponse cellulaire.

Des séquences d'acides aminés exogènes préférées de l'invention, contiennent des séquences peptidiques respectivement caractéristiques des virus SIV, HIV-2, HIV-1 et ont les séquences suivantes :

RGEFLYCKMNWFLNWVEDRSLTTQKPKERHKRNYVPCHIRQIINTWHKVGKNVYLP PREGDLTCNSTVTSLIANINWTDG

RGEFLYCNMTWFLNWIENKTHRNYAPCHIKQIINTWHKVGRNVYLPPREGELSCNS TVTSIIANIDWQNN

GGEFFYCNSTQLFNSTWFNSTWSTEGSNNTEGSDTITLPCRIKQFINMWQEVGKAM YAPPISGQIRCSSNITGLLLTRDGGNN

D'autres séquences peptidiques exogènes peuvent entrer dans le cadre de l'invention dès lors que les propriétés recherchées pour les particules recombinantes et qui ont été précédemment définies, sont respectées.

Les peptides et polypeptides immunogènes caractéristiques de HIV ou de SIV qui entrent dans le cadre de l'invention peuvent être obtenus par synthèse selon les techniques décrites dans le brevet US 4629783 délivré le 16 Décembre 1986 à la société GENETIC SYSTEMS.

D'autres méthodes dont les principes sont résumés ci-après, pourront être mises en oeuvre.

Un principe de synthèse est par exemple de mettre en présence une phase solide dans laquelle s'assemble la séquence du peptide et une phase liquide contenant solvants et réactifs. A chaque étape la séparation entre le peptide en croissance et les réactifs se fait par simples filtrations et lavages.

Initialement, le support solide qui porte un groupement réactif réagit avec le carboxyle d'un acide aminé introduit avec son $\alpha$-NH$_2$ bloqué (protégé par exemple par le t-butyloxy carbonyle), pour établir une liaison covalente. Après déprotection de la fonction aminée (par exemple en lavant avec un acide tel que l'acide trifluoroacétique), un deuxième acide aminé protégé est introduit pour former par couplage la première liaison peptidique. Le deuxième acide aminé, qui fournit le deuxième amino-acyle de la séquence, est couplé à partir du résidu amino-acyle C-terminal sur la fonction amine déprotégée du premier acide aminé C-terminal de la chaîne. De préférence la fonction carboxyle de ce deuxième acide aminé est activée, par exemple par dicyclohexylcarbodiimide. Par une suite de réactions, déprotections et couplages, la synthèse progresse du résidu C- vers le résidu N- terminal. Lorsque la séquence voulue a été assemblée le peptide est décroché de la phase solide, par une réaction spécifique de coupure de la liaison peptide-résine établie initialement.

Selon un autre mode de réalisation de l'invention, ou aura recours à la technique de synthèse en solution homogène décrit par HOUBENWEYL dans l'ouvrage intitulé "Méthode der Organischen Chemie" (Méthode de la Chimie Organique) édité par E. Wunsch, vol. 15-I et II, THIEME, Stuttgart 1974.

Cette méthode de synthèse consiste à condenser successivement deux-à-deux les aminoacyles successifs dans l'ordre requis, ou à condenser des aminoacyles et des fragments préalablement formés et contenant déjà plusieurs aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments, à l'exception des fonctions amines de l'un et carboxyles de l'autre ou vice-versa, qui doivent normalement intervenir dans la formation des liaisons peptidiques, notamment après activation de la fonction carboxyle, selon les méthodes bien connues dans la synthèse des peptides. En variante, on pourra avoir recours à des réactions de couplage mettant en jeu des réactifs de couplage classique, du type carbodiimide, tels que par exemple la 1-ethyl-3-(3-diméthyl-aminopropyl) -cdarbodiimide. Lorsque l'aminoacyle mis en oeuvre possède une fonction acide supplémentaire (notamment dans le cas de l'acide glutamique), ces fonctions seront par exemple protégées par des groupes t-butylester.

L'invention se rapporte aussi à un hôte cellulaire transformé par un vecteur recombinant parmi ceux qui ont été précédemment décrits et compétents pour assurer l'expression de l'insérat contenu dans ce vecteur recombinant.

A titre d'exemple, l'expression de la particule hybride recombinante HIV-HBs peut être assurée dans différents types cellulaires par des vecteurs plasmidiques ou viraux, par exemple les pox virus (du type du virus de la vaccine, les adénovirus, les baculo virus). Les techniques d'infection ou de transfection selon qu'il s'agit de virus ou de plasmide sont bien connues de l'homme de l'art. On pourra pour cela se reporter au brevet français n° 245.136 ou à la demande de brevet européen publiée sous le n° 265.785.

A titre d'exemple d'hôtes cellulaires adaptés pour la réalisation de l'invention, on mentionne des cellules eucaryotes de mammifère par exemple des cellules CHO, ou encore des levures, par exemple <u>Saccharomyces cerevisiae</u>

Les procédés de transformation mis en oeuvre pour obtenir l'expression de l'insérat du vecteur (et de mise en évidence du produit d'expression) sont en eux-mêmes classiques, comme il résulte des exemples qui suivent.

L'invention vise par ailleurs une composition vaccinante contre des infections dues à un rétrovirus HIV, caractérisée en ce qu'elle comprend en tant que principe actif, des particules recombinantes hybrides telles décrites dans les pages précédentes, en association avec un véhicule pharmaceutique acceptable.

L'administration de ces compositions à un patient pourra être faite en utilisant des doses contenant d'environ 5 à environ 20 $\mu$g de particules recombinantes hybrides purifiées par dose. Ces doses seront par exemple administrées trois fois à un mois d'intervalle.

L'invention concerne également un procédé de préparation d'un vecteur recombinant, caractérisé par les étapes suivantes :

- la recombinaison génétique <u>in vitro</u> le cas échéant sous le contrôle d'un promoteur reconnu par les polymérases d'un hôte cellulaire eucaryote approprié, d'une part d'une séquence nucléotidique exogène codant pour une séquence d'acides aminés immunogène, induisant des anticorps neutralisants vis-à-vis d'un rétrovirus de la famille de HIV ou susceptible d'être reconnue de tels anticorps, cette séquence exogène comprenant au moins un épitope susceptible d'être reconnu par les lymphocytes B, et au moins un épitope susceptible d'être reconnu par les lymphocytes T, et d'autre part, un enchaînement de nucléotides correspondant à la région S et le cas échéant à tout ou partie de la région pré-S du génome du virus de l'hépatite B et

- la récupération du vecteur recombinant formé comprenant les éléments tels qu'ils ont été définis plus haut.

Un procédé semblable peut être appliqué pour obtenir la formation d'un vecteur recombinant précédemment défini, dans lequel la séquence exogène insérée code pour une séquence d'acides aminés comprenant en outre un site d'attachement au récepteur CD4.

La récupération du vecteur recombinant peut être réalisée par les procédés connus, notamment par hybridation sélective avec, d'une part, des sondes nucléotidiques caractéristiques des séquences correspondantes du génome d'un rétrovirus HIV, d'autre part, avec des sondes caractéristiques de la région S du génome du virus de l'hépatite B.

L'invention se rapporte aussi à des compositions pour le diagnostic in vitro d'une infection due à HIV par détection de la présence d'anticorps neutralisants dans un milieu biologique d'un patient. De telles compositions comprennent des particules hybrides recombinantes exprimant à leur surface un ou plusieurs des polypeptides ou peptides de HIV-1 ou HIV-2 ou SIV, ou un mélange de ces particules hybrides recombinantes.

L'invention concerne aussi l'utilisation de ces particules hybrides recombinantes pour réaliser un test de diagnostic in vitro de la présence d'anticorps résultants d'une infection par HIV caractérisé par :

- la mise en contact d'un milieu biologique d'un patient avec des particules recombinantes hybrides,
- la détection du produit de réaction éventuellement formé à savoir un complexe antigène-anticorps.

Les moyens de détection du complexe antigène-anticorps sont ceux connus de l'homme de l'art notamment le marquage radioactif. Le test pourra, selon la composition des particules hybrides recombinantes, être appliqué pour le diagnostic sélectif de HIV-1 ou HIV-2, ou pour le diagnostic non sélectif grâce à l'utilisation de compositions comprenant des particules exprimant à leur surface des séquences de HIV-1 et de HIV-2 ou une séquence caractéristique des deux virus à la fois.

De même l'invention concerne un procédé pour le diagnostic in vitro d'une infection due à HIV, caractérisé par les étapes suivantes :

- mise en contact d'une composition ci-dessus définie avec un milieu biologique d'un patient susceptible de contenir des anticorps neutralisants contre HIV,
- détection de la formation d'un éventuel complexe antigène-anticorps.

Par milieu biologique on entend tout échantillon susceptible de contenir des anticorps, tel que le sérum d'un patient.

L'invention vise encore un procédé pour la production des particules hybrides recombinantes ci-dessus, caractérisé par les étapes suivantes :

- la transformation d'un hôte cellulaire déterminé avec un vecteur recombinant tel que décrit ci-dessus, dont le promoteur éventuellement présent dans le vecteur est reconnu par les polymérases de l'hôte cellulaire transformé,
- la culture de l'hôte cellulaire sur un milieu approprié et la récupération des particules formées, plus particulièrement celles excrétées dans le milieu de culture. Ces particules sont alors caractérisables non seulement par leur caractéristiques morphologiques et leur réactivité immunologique avec des anticorps anti-HBs, mais également par leur aptitude à être reconnues par les anticorps anti-HIV correspondants ou à induire de tels anticorps.

L'invention vise également l'utilisation des fragments immunogènes d'acides aminés décrits précédemment, seuls ou en mélanges, en tant qu'antigènes d'immuno-essais pour la détection in vitro d'anticorps, en particulier d'anticorps neutralisants, notamment en suivi de vaccination.

L'invention concerne donc également une méthode de dosage in vitro de la présence d'anticorps dirigés contre un rétrovirus du type HIV, en particulier pour le dosage d'anticorps neutralisants en suivi de vaccination, comprenant les étapes suivantes :

- la mise en contact des fragments immunogènes d'acides aminés décrits, marqués de façon à permettre le dosage ultérieur, avec le milieu biologique à tester, dans des conditions appropriées pour permettre la réaction entre lesdits fragments et le milieu,
- le dosage desdits fragments ayant réagi avec le milieu.

L'invention vise également une trousse (un kit) pour le diagnostic in vitro d'anticorps anti-HIV, dans un milieu biologique, en particulier d'anticorps neutralisants en suivi de vaccination, caractérisée en ce qu'elle comprend :

- des fragments immunogènes d'acides aminés tels que décrits ci-dessus,
- des moyens adaptés pour la révélation de la réaction antigène-anticorps.

Des moyens de révélation consistent par exemple en des anti-anticorps capables de reconnaître le conjugué formé entre les antigènes et les anticorps du milieu biologique.

D'autres caractéristiques et avantages particulières de l'invention apparaîtront dans les exemples qui suivent et qui sont illustrés par les figures.

MATERIELS ET METHODES

Construction de vecteur

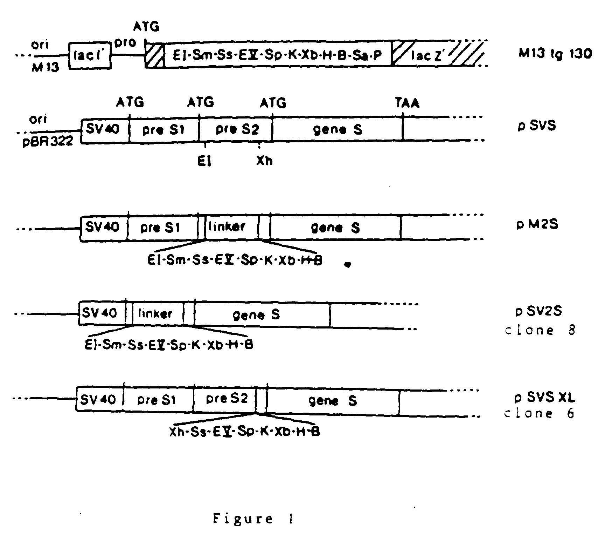

M13tg130 est un vecteur de clonage dérivé du bactériophage M13. Comme l'on décrit KIENY et al (14), il contient dans la séquence 5′ du gène codant pour la beta-galactosidase, 11 sites uniques de restriction constituant un polyadaptateur (polylinker) (voir figure 1). L'insertion de fragments d'ADN exogène dans ce "polylinker" peut être détectée par absence de coloration par X-gal. Le criblage des plaques bleues par hybridation in situ permet la détection des fragments d'ADN insérés en phase.

Le vecteur d'expression de HBsAg, pSVS (11) a été modifié pour y insérer le segment EcoRI -SalI de l'ADN de M13tg130 contenant 10 sites de restriction entre les sites EcorRI et XhoI de la région pré-S2 du génome du virus de l'hépatite B encore désigné par HBV (abréviation anglaise de Hépatitis B virus). La fusion entre les sites de restriction SalI et XhoI restaure le cadre de lecture dans la région pré-S2. La région pré-S1 du vecteur résultant pM2S a ensuite été délétée. Le vecteur obtenu a été désigné par pSV2S. Dans cette construction, la fusion entre l'ADN cloné et le gène S adjacent à la région pré S2 est placée sous le contrôle direct du promoteur précoce de SV40.

En utilisant des moyens analogues, on a obtenu un vecteur désigné par pSVS XL par recombinaison entre pSVS et pM2S au niveau des sites XhoI etSmaI respectivement.

Transfection des cellules animales

Des cellules d'ovaire de hamster chinois dhfr⁻ (CHO) du clone DXB11 (15) ont été maintenues sous forme de monocouche dans un milieu Ham F12 complété avec 10 % de sérum de veau foetal. Les cellules ont été tranfectées en utilisant la méthode au phosphate de calcium décrite dans la référence (16) avec un choc au glycérol 6 heures plus tard (Parker et al.1979). Une cotransfection avec le vecteur pSV2 dhfr selon le principe rappelé dans la référence (17) permet la sélection des clones stables exprimant les protéines de fusion avec HbsAg(encore appelées protéines recombinantes). L'amplification des séquences transformantes a été obtenue en procédant à une culture cellulaire en présence de concentrations croissantes de méthothréxate (MTX) (18).

Production et purification des particules recombinantes de HIV-HBsAg.

Des clones stables exprimant les particules recombinantes de HBsAg ont été cultivées jusqu'à confluence et ont été alimentés tous les trois ou quatre jours avec un milieu de culture contenant seulement 5 % de sérum de veau foetal. Les surnageants de cultures cellulaires ont été récupérés, clarifiés et les particules de HBsAg ont été concentrées par précipitation par le sulfate d'ammonium et purifiées à deux reprises dans un gradient isopycnique de CsCl suivi par un gradient de vélocité en sucrose de 0 à 20 %.

Immunoblots

1 µg environ des particules recombinantes purifiées a été soumis à ébullition dans un tampon de Laemmeli, séparé par électrophorèse sur gel de polyacrylamide dodecyl sulfate de sodium 12,5% (NaDodSO$_4$) et éléctrotransféré sur des filtres de nitrocellulose (SCHLEICHER & SCHULL).

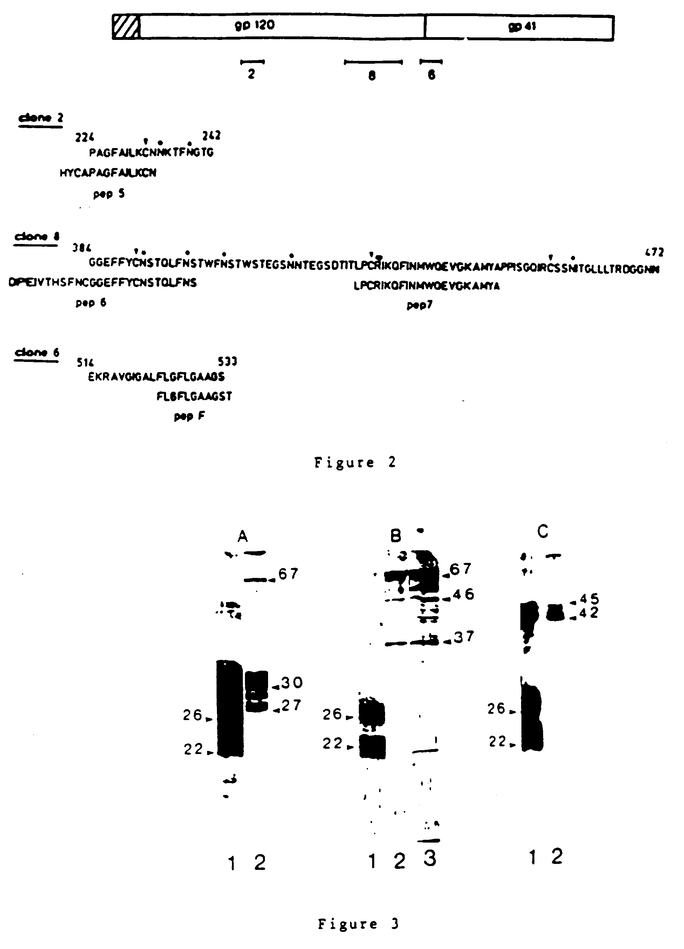

Les protéines immobilisées sur les filtres ont réagi avec les différents antiséra suivants : a-p49a (19), antisérum, anti-peptide de lapin spécifique de HBsAg, 18/7 (20) anticorps spécifique de la région pré-S1 de HBV et un antisérum dirigé contre les régions de la GP160 de HIV1 (figure 2) obtenu à partir de lapins en utilisant des peptides synthétiques couplés avec un sérum albumine bovine (BSA : abréviation anglaise de "bovine serum albumine") ou l'hémocyanine limpet keyhole (abréviation anglaise : KLH).

Les protéines immunoréactives finales ont été détectées par des anticorps anti-lapin ou par des immunoglobulines de souris conjuguées avec de la peroxydase (AMERSHAM) et ont été révélés avec la diaminobenzidine.

Immunisations et mesures de la production d'anticorps

Des lapins ont été immunisés deux ou trois fois à un mois d'intervalle par injection intradermique d'environ 4 µg de particules recombinantes de HBsAg purifiées (tableau 1) dans l'adjuvant complet de Freund. Des séra ont été obtenus 30 jours après la dernière injection.

Ces antiséra ont été testés pour connaître leur niveau de production d'anticorps, par la méthode ELISA (Enzyme Linked Immunoabsorbent Assay), en utilisant, en phase solide, des particules HBsAg dérivées du plasma (Hevac B.Pasteur, produit par Pasteur Vaccins France) ou des peptides synthétiques caractéristiques de HIV (voir figure 2). Les peptides synthétiques ont été fournis par Monsieur le Dr ROCHAT (UA. 553, CNRS MARSEILLE). Tous les antigènes ont été utilisés à une concentration de 1 µg/ml sur des plaques de microtitrage (FALCON "Probind") et les tests ELISA ont été réalisés comme décrit par CHARBIT et al (21).

Essais sur les anticorps neutralisants

Pour mettre en évidence l'activité neutralisante des anticorps contenus dans les sera, on a utilisé des lymphocytes stimulés du sang phériphérique humain (PBL). Ces cellules ont été utilisées pour leur sensibilité à l'infection à HIV. Différents échantillons d'un isolat $LAV_{BRU}$ décrit par MONTAGNIER et al (22) ont été utilisés comme inoculum de virus. Chaque dilution de sérum a été pré-incubée avec un volume égal d'inoculum viral pendant 1 heure à 37°C. Le mélange a ensuite été ajouté à une quantité de $4.10^6$ PBL humains dans un milieu RPMI 16-40 complété avec 10 % de sérum de veau foetal contenant 20 U./ml d'interleukine, 2,2 µg/ml de polybrène et 25 U./ml de séra anti-interféron.

Après 1 heure d'incubation à 37°C, les cellules ont été rincées et cultivées dans des milieux RPMI complets. Les surnageants ont été retirés deux fois par semaine et les mêmes dilutions des séra testés ont été ajoutées dans les nouveaux milieux les deux premières fois. Les surnageants ont été congelés à -70°C et testés pour connaître leur activité reverse-transcriptase (activité RT) (23).

RESULTATS

Clonage des fragments du gène d'enveloppe de HIV dans un vecteur d'expression eucaryote de HBsAg

Le clonage a été réalisé en deux étapes :

Dans une première étape, on a construit une banque génomique d'expression, dans le bactériophage M13tg130 et les fragments d'ADN sélectionnés ont été transférés dans le vecteur d'expression d'HBsAg. Le bactériophage M13tg130 est un vecteur de clonage et de séquençage portant au niveau du cinquième codon du gène lacZ de E. coli, un polylinker (polyadaptateur) contenant 11 sites de restriction uniques. Des fragments d'ADN du génome de HIV coupés au hasard et comprenant de 50 à 1000 paires de base, ont été clonés dans les sites Smal et EcoRV du bactériophage M13tg130 (figure 1). L'hybridation in situ et le séquençage ont permis de repérér précisément le fragment cloné dans la région codant pour l'enveloppe de HIV1.

Les fragments de HIV sélectionnés ont été transférés dans le vecteur d'expression de HBsAg, pSV2S (figure 1). pSV2S est obtenu par modification d'un vecteur d'expression pSVS précédemment décrit dans la partie "Matériels et Méthodes". pSVS comprend la séquence de SV40 contenant l'origine de réplication, les promoteurs tardif et précoce, l'activateur et les sites d'initiation de l'$ARN_m$. On trouve au voisinage immédiat du promoteur précoce, des séquences de HBV contenant la région génomique pré-S2, le gène S, et une séquence permettant la polyadénylation, de l'ARN messager de l'HBsAg. Dans le vecteur pSV2S, la partie de la région pré-S2 a été délétée et remplacée par le polyadaptateur M13tg130. Dans cette construction, le polyadaptateur permet l'insertion d'un fragment d'ADN en phase avec la région pré-S2 et le gène S. Ainsi, tout fragment cloné dans le cadre de lecture correct du gène LacZ dans le bactériophage M13tg130 peut être facilement transféré en phase avec le gène S dans le vecteur d'expression pSV2S.

Un autre dérivé de pSVS est le vecteur pSVS XL dans lequel l'adaptateur M13tg130 permet aisément l'insertion en phase d'une séquence exogène dans la région pré-S2. Dans cette construction, les antigènes pré-S1 et pré-S2 sont exprimés de façon adjaçente avec l'antigène étranger.

Parmi les fragments correspondant à l'enveloppe de HIV et obtenus dans la banque, trois ont été étudiés en détail.

Le clone n°2 contient un insérat de HIV formé de 52 paires de bases (bp) (nucléotides 6440 à 6491), situé dans la partie médiane dans la séquence codant pour la GP120 (figure 2). Il couvre une région conservée entre les différents isolats de HIV1 (3).

Le clone n°8 contient un insérat de HIV de 252 paires de bases (nucléotides 6915 à 7166), correspondant à la partie sub-carboxyterminale de la protéine GP120 (figure 2). Cette région est relativement bien conservée parmi les différents isolats et présente une homologie avec la région constante de la chaîne lourde de l'immunoglobuline (référence 24). De plus, une partie du fragment d'ADN code pour un domaine impliqué dans l'attachement de la protéine GP120 au récepteur du virus, la molécule CD4 (référence 25).

Le clone n°6 contient un insérat de HIV ayant 55 paires de bases (nucléotides 7306 à 7360) situé dans une région codant pour la partie amino-terminale de la protéine transmembranaire GP41. Cette région est conservée dans les isolats de HIV et partage une homologie avec la protéine de fusion d'autres virus (26).

Expression des protéines de fusion HIV-HBV dans les cellules transfectées

Les vecteurs d'expression de HBsAg contenant les fragments du gène d'enveloppe de HIV-1 ont été utilisés pour transfecter des cellules CHO. Les clones stables exprimant HBsAg, détectés par un test RIA spécifique pour la protéine majeure d'enveloppe d'HBV ont été criblés pour détecter l'expression du gène hétérologue. Ils ont ensuite été caractérisés par analyse selon la méthode dite "Northern blot" et l'ARN messager hybride a été observé. Les particules secrétées ont ensuite été purifées à partir du surnageant de culture cellulaire par un procédé utilisé pour les particules de HBsAg (11). Les particules recombinantes HIV-HBsAg ont à peu près la même densité dans un gradient CsCl que les particules natives. Les protéines des particules recombinantes ont été séparées par électrophorèse sur gel de polyacrylamide NaDodSO$_4$, transférées sur des filtres de nitrocellulose et testées avec des antiséra obtenus à partir de lapin et dirigés contre le peptide synthétique correspondant soit à la particule HBsAg soit aux déterminants de l'enveloppe de HIV (figure 3).

Les résultats suivants ont été observés :

Particule recombinante n°2 :

en plus de la protéine majeure HBsAg et de la protéine correspondante sous forme glycosylée GP26, le sérum anti-p49a a révèlé 4 bandes dans l'intervalle de 27 000 à 32 000 daltons correspondant à la protéine moyenne (figure 3A, ligne 1). les 4 bandes sont également fortement révélées par un sérum dirigé contre le peptide de HIV1 homologue (pep 5) (figure 3A, ligne 1). Ceci a montré que la particule recombinante HBsAg n°2 porte un déterminant étranger. Les quantités de protéines moyenne et majeure sont pratiquement les mêmes. La protéine moyenne fusionnée est présente sous quatre formes, en fonction de la glycosylation des régions de HBV (1 site de glycosylation) et de HIV (2 sites possibles de glycosylation).

Particule recombinante n°8 :

Le sérum anti-p49a a révèlé une protéine majeure de HBsAg sous deux formes (P22 et GP26) ainsi que des bandes correspondant à un poids moléculaire s'étendant de 37 000 à 46 000 (figure 3B, ligne 1). Des séra dirigés contre deux peptides homologues n° 6 et n° 7 ont révélé la même répartition de protéines lourdes (figure 3B, lignes 2 et 3) démontrant la présence d'un déterminant de HIV dans les particules. La quantité de protéine moyenne fusionnée est moins importante que la quantité de protéine majeure. La protéine de 37 kd correspond à la protéine fusion non glycosylée et les protéines de plus de haut poids moléculaire correspondent aux formes glycosylées (5 sites possibles de glycosylation dans la partie HIV). Les séra anti-peptides ont également révélé une protéine de 67 kd qui correspond à la sérum-albumine co-purifiée.

Particule recombinante n°6 :

le sérum anti-p49a a révélé une protéine majeure HBsAg et sa contrepartie glycosylée mais n'a pas révélé de protéine moyenne (figure 3C, ligne 1). Un triplet de bandes de hauts poids moléculaires a été observé ; il est aussi révélé par un anticorps monoclonal contre l'antigène pré-S1 (figure 3C, ligne 2). La taille de ces protéines est compatible avec celle de la grande protéine HBsAg, comprenant le peptide étranger dans ses formes glysosylée et non glycosylée.

Réponse immunitaire humorale à des particules recombinantes HBsAg

Des lapins ont été immunisés deux ou trois fois à un mois d'intervalle, avec chacune des particules recombinantes HBsAg ci-dessus, dans un adjuvant de Freund complet (voir "Matériels et Méthodes"). La spécificité de la réponse immunitaire a été déterminée par un test ELISA en utilisant des particules dérivées du plasma et différents peptides correspondants de HIV1. Des antiséra ont également été testés contre les peptides pré-S2 (séquence d'acides aminés de la protéine d'enveloppe de l'HBV comprise entre les numéros 120 à 145 inclus) (tableau 1).

Tous les lapins ont développé des anticorps contre les particules HBsAg natives, à différents niveaux. La plus faible réponse a été obtenue pour le recombinant n° 8, ce qui peut s'expliquer par la taille relativement grande de l'antigène étranger (84 acides aminés) qui peut entrer en compétition avec les déterminants HBsAg ou les masquer. Au contraire, la meilleure réponse dirigée contre le peptide étranger a été obtenue avec ce recombinant n° 8, ce qui suggère dans ce cas une présentation prédominante des déterminants antigéniques HIV vis-à-vis des déterminants antigéniques de HBsAg.

Dans les recombinants n° 2 et n°6, les antigènes exogènes appartenant à HIV sont plus petits et de taille comparable (18 acides aminés et 20 acides aminés). Les réponses dirigées contre les peptides observés dans les deux cas sont plus faibles que celles dirigées contre les particules HBsAg natives. Le taux de protéine de fusion vis-à-vis de la protéine majeure native est élevé dans le recombinant n°2 (voir figure 3). Ces résultats suggèrent une faible immunogénicité de la séquence insérée dans ce recombinant. Une réponse avec les peptides pré-S2 est obtenue uniquement pour le recombinant n°6 résultant de l'expression dans le vecteur pSV XL (figure 1). Des antiséra des deux autres lapins immunisés avec des particules obtenues par expression dans le vecteur pSV2S sont négatives et servent de contrôle.

<u>Neutralisation de l'infection due à HIV in vitro par des séra immmuns dirigés contre les particules recombinantes n°8</u>

Des lapins ont été immunisés avec des particules recombinantes HIV-HBsAg et les séra ont été testés pour connaître leur capacité à neutraliser l'isolat $LAV1_{BRU}$ (24). L'infection virale dans les lymphocytes du sang périphérique a été étudiée grâce à l'activité reverse-transcriptase (RT) pendant trois à quinze jours. L'essai réalisé est fondé sur la réduction de l'activité RT en présence de sérum immun, le résultat étant comparé au résultat obtenu en présence de sérum pré-immun du même lapin.

Dans ce test, les anticorps ne peuvent pas neutraliser efficacement le virus si des quantités saturantes de virions infectieux sont présentes.

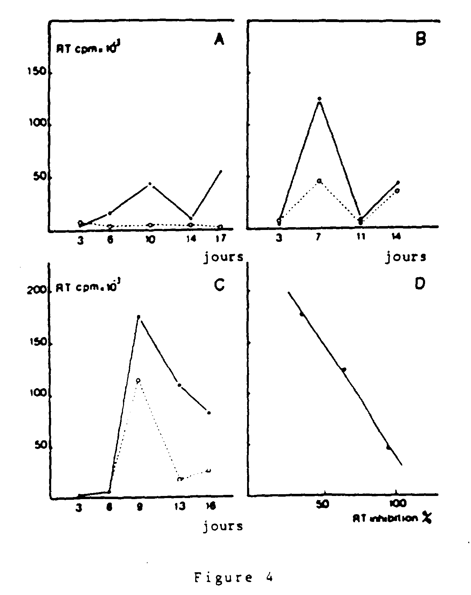

C'est pourquoi les tests ont été réalisés à partir des différents antiséra dilués (1:50) pour neutraliser les différents échantillons de virus (figure 4A, 4B, 4C). Le sérum du lapin immunisé avec la particule recombinante n°8 a neutralisé intégralement l'infectivité virale du virus le moins infectieux (figure 4) et on a observé une diminution de l'activité RT de 95 % à 63 % et 35 % lorsque l'on a augmenté l'infectivité de l'échantillon viral (figure 4D). Ceci montre que la capacité de neutralisation des antiséra est fonction de la quantité de virus.

Des antiséra correspondant aux particules recombinantes n°6 ont donné une neutralisation faible à une faible concentration de virus mais on n'a pas pu détecter une réduction de l'activité RT avec l'antisérum de lapin immunisé avec le recombinant n° 2, même à une faible concentration de virus.

De plus, la neutralisation de différents isolats de HIV1 a été examinée en utilisant une concentration constante de virus avec une dilution variable des antiséra dirigés contre la particule recombinante n°8.

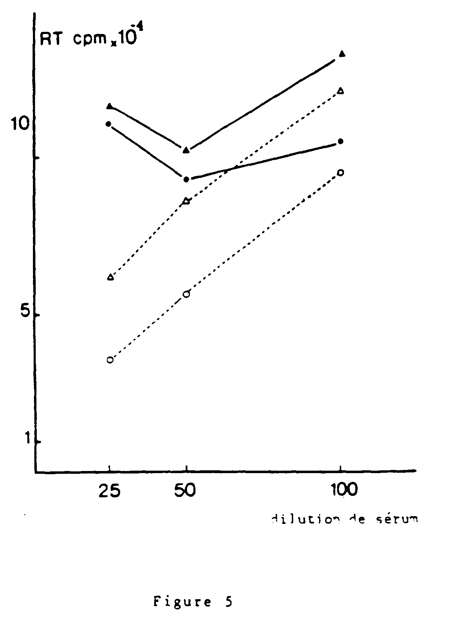

La figure 5 montre une réduction de 50 % de la capacité infectieuse de l'isolat $LAV_{BRU}$ avec une dilution 1/50 comparée au sérum pré-immun.

L'activité neutralisante de cet anti-sérum contre l'isolat zaïrois de HIV1 a également été montrée : un effet neutralisant de 50 % a été observé à une dilution 1/25, sur $LAV_{ELI}$ (3) (figure 5).

DISCUSSION

la glycoprotéine interne d'enveloppe de HIV1, GP120, a priori intéressante pour sa capacité à induire une réponse immunitaire protectrice vis-à-vis de HIV, contient des régions conservées et d'autres régions variables entre les différents isolats de HIV. Les séquences conservées à l'intérieur des domaines constants pourraient correspondre à des régions de séquences ayant une importance fonctionnelle potentielle. Comme le montrent les résultats rapportés plus haut, ces différentes régions conservées de l'enveloppe de HIV1 ont été choisies et exprimées sous forme de protéines de fusion avec la protéine moyenne de l'antigène de surface de l'hépatite B.

La particule HBsAg présente de bonnes propriétés immunogènes selon deux aspects exploités par les inventeurs :

i) c'est un polymère de haut poids moléculaire portant à sa surface de nombreuses copies de déterminants antigéniques ;

ii) elle porte également des épitopes T qui peuvent être utiles dans la reconnaissance d'épitopes B étrangers par le système immunitaire (27).

Parmi les trois différentes régions conservées de HIV étudiées plus haut, l'une d'entre elles (clone n°8) est liée au site de liaison CD4 (25), une autre (clone n°6) pourrait être impliquée dans le processus de fusion (26), et la troisième (clone n°2) n'est reliée à aucune activité biologique connue.

Les particules recombinantes HIV-HBsAg obtenues par fusion de ces déterminants antigéniques avec le produit du gène de la région pré-S2 ont été étudiées en utilisant des anticorps dirigés contre des peptides homologues synthétiques. Des analyses Western-blot ont permis de mettre en évidence la présence des déterminants antigéniques HIV sur les particules et la glycosylation probable des protéines de fusion. Les taux de protéine de fusion et de protéine native majeure observés dans les différentes particules recombinantes sont variables. Pour le recombinant n°8, la protéine de fusion est apparemment moins abondante, ce qui peut être lié à la taille relativement importante de la séquence étrangère insérée. La taille d'origine du produit de traduction pré-S2 de HBV est de 55 acides aminés alors que la construction de protéine de fusion augmente cette taille jusqu'à 111 acides aminés. La taille de la protéine à fusionner pourrait contrarier son insertion dans les particules de HBsAg.

Pour les recombinants n°6 et n°2, le taux entre la protéine de fusion et la protéine majeure est comparable à celui observé entre la protéine moyenne et la protéine majeure dans les particules obtenues par transfection du vecteur d'expression pSVS des particules HBsAg d'origine (11).

L'immunogénicité des particules recombinantes HIV-HBsAg a été rapportée plus haut. La réponse humorale est dirigée contre deux régions antigéniques et montre que les épitopes étrangers sont présents de façon efficace à la surface des particules.

L'activité biologique des anticorps a été testée lors d'essais de neutralisation in vitro. Des épitopes neutralisants et non-neutralisants ont été mis en évidence dans les protéines d'enveloppe de HIV. Des anticorps dirigés contre les recombinants n°8 ont un effet neutralisant potentiel sur l'isolat HIV (LAV$_{BRU}$) utilisé à l'origine pour fabriquer des protéines de fusion. Un effet plus faible a été observé lorsque les anticorps ont réagi avec un isolat de HIV1 différent (LAV$_{ELI}$). L'insérat de HIV utilisé dans le recombinant n°8 couvre deux régions relativement bien conservées parmi les différents isolats HIV1, ces deux régions étant séparées par une région hypervariable caractéristique de l'isolat. Dans cette région, CEASE et al ont identifié un épitope de cellule T (30).

De plus, des expériences faisant appel à des anticorps monoclonaux dirigés contre la GP120 ainsi que des expériences de mutagénèse in vitro ont permis d'identifier une séquence importante pour l'interaction avec le récepteur CD4 (25). On sait également qu'une protéine de fusion a été produite dans E. coli portant une séquence chevauchant en partie la séquence précédemment utilisée et capable de conduire à la formation d'anticorps neutralisants et à une réponse immunitaire cellulaire (31). Dans le système HBsAg, les protéines de fusion peuvent être glycosylées normalement, ce qui peut être un élément important pour la production d'anticorps ayant une forte affinité vis-à-vis des épitopes et capables de bloquer l'interaction de la protéine d'enveloppe de HIV et du récepteur CD4. De plus, le déterminant antigénique de HIV semble mieux exposé à la surface des particules que dans la protéine virale native GP. L'efficacité inférieure de la neutralisation observée avec l'isolat zaïrois LAV$_{ELI}$ peut être attribuée à la variation de la séquence d'aminoacides d'environ 30 % dans une région déterminante pour l'interaction entre le virus et le récepteur CD4 (25).

Les anticorps dirigés contre les recombinants n°2 et n°6 ont également été testés dans des expériences de neutralisation. Les séquences de HIV portant le recombinant n°2 n'ont pas induit d'anticorps neutralisants lors de ces expériences. Une réponse immunitaire faible des animaux utilisés ou une faible immunogénicité de la région impliquée dans le recombinant n°2, pourrait expliquer ce résultat. La partie amino-terminale de la GP41 pourrait être impliquée dans le processus de fusion et en conséquence dans la neutralisation virale.

De même, l'effet potentiel des anticorps obtenus par immunisation avec le recombinant n°6 vis-à-vis de l'infectivité de HIV1 a été testée. L'effet neutralisant est faible et l'inhibition de la formation des syncitia n'est pas complète. Ce résultat pourrait être attribué à à la taille limitée de l'insérat de HIV.

Les inventeurs ont donc réalisé un système potentiellement capable de permettre la présentation des déterminants antigéniques étrangers de l'enveloppe de HIV, ce système permettant en outre la production d'anticorps neutralisants dirigés contre une région de la protéine d'enveloppe qui présenterait sous forme native des propriétés immunogéniques faibles ou ne serait pas présentée à la surface dans cette protéine native.

Par ailleurs, la juxtaposition de déterminants antigéniques de HBV et de HIV à la surface de la même particule immunogénique peut permettre la réalisation de vaccins polyvalents.

## IMMUNISATION DE MACAQUES AVEC DES PARTICULES HIV/HBsAg

Les particules obtenues de la façon précédemment décrite en fusionnant une séquence de 84 aminoacides (AA 384 à 472) de la glycoprotéine externe d'enveloppe de HIV-1 à l'intérieur de la partie pré-S2 de la protéine centrale HBsAg ont été utilisées dans un test chez des macaques pour tester la production d'anticorps neutralisants.

Pour montrer que ces particules hybrides induisent une réponse humorale aussi bien qu'une réponse immune médiée par des cellules T, vis-à-vis de HIV, chez les primates, des macaques ont été immunisés avec soit des particules hybrides, soit des particules HBsAg natives.

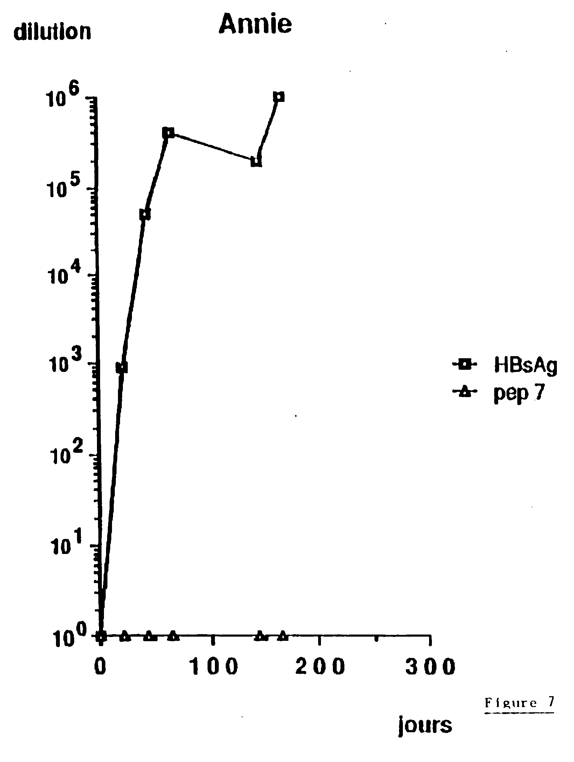

Deux macaques ont été immunisés avec des particules purifiées HIV/HBsAg, par 3 injections sous-cutanées à 1 mois d'intervalle. Ces macaques ont ensuite subi un rappel à deux reprises trois mois plus tard. Un macaque contrôle a été immunisé avec 4 doses de particules HBsAg natives, suivant le même protocole. Des échantillons de sang ont été prélevés chez chaque animal avant et après chaque immunisation. La réponse immune humorale a été suivie avec un test ELISA (figure 7) en utilisant soit les particules natives HBsAg ou les peptides synthétiques homologues de HIV (peptide 6:AA 373-398, peptide 7:AA 421-441, voir figure 3) sur phase solide.

Les titres anti-HBs ont présenté un pic 3 semaines après la troisième dose de vaccin et ensuite une chute. Un second pic des titres a été observé après l'immunisation de rappel sauf chez le singe Mac S dont le titre d'anticorps est resté constant après la quatrième injection, et a crû lentement après la 5ème injection. Des anticorps anti-HIV ont été détectés au moyen d'un test ELISA en utilisant les deux peptides. Les titres ont atteint un niveau maximal trois semaines après la deuxième injection (Mac H) ou après la troisième injection (Mac S) et ont ensuite chuté lentement. Toutefois, la réponse anti-HIV semble atteindre un plateau après la seconde injection.

De la même façon que pour les titres anti-HBs, un effet de rappel de la 4ème dose est détecté avec le sérum de Mac H.

Chez le singe contrôle Mac A immunisé avec des particules natives HBsAg, un titre en anticorps anti-HBs élevé après la 3ème injection avec une croissance après la dose de rappel a été détecté. Aucun anticorps anti-HIV n'a été détecté en faisant réagir les peptides de HIV avec l'un quelconque des sera de cet animal de contrôle.

Ces études montrent que l'immunisation avec deux doses des particules hybrides semble suffisante pour établir une mémoire immunologique et que les doses de rappel sont nécessaires pour maintenir le titre élevé de la réponse immune.

Pour déterminer si une réponse immune par l'intermédiaire des cellules T à l'égard des deux parties de la particule hybride pouvait être détectée chez les singes vaccinés, des lymphocytes de sang périphérique (PBL) ont été testés pour leur capacité à proliférer et à incorporer de la thymidine $^3$H en réponse à une stimulation avec des particules HBsAg natives ou avec des particules HIV/HBsAg ainsi qu'avec du HIV purifié inactivé par chauffage. En tant que contrôle interne de la prolifération des PBL, une concanavaline A mitogène a été utilisée. Les PBL de chaque macaque ont été isolés avant l'immunisation et 21 jours après la seconde et la troisième injection, ainsi que avant et après le rappel avec les particules HBsAg. Ils ont été cultivés dans un milieu seul ou dans un milieu stimulé pendant 4 jours avec HBsAg ou Con A et pendant 5 jours avec HIV-1.

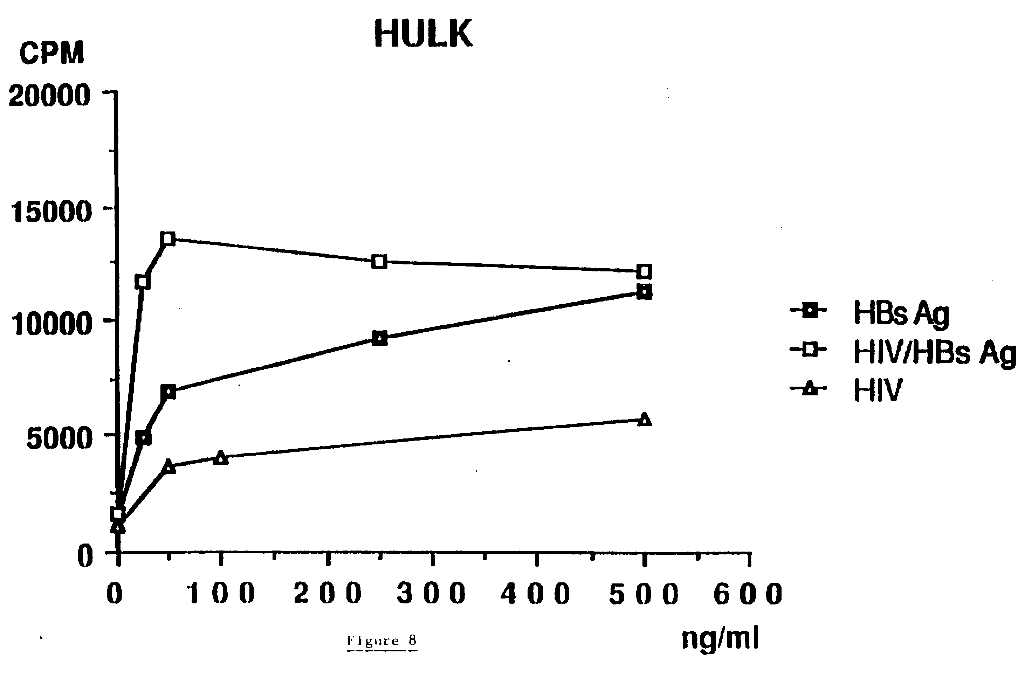

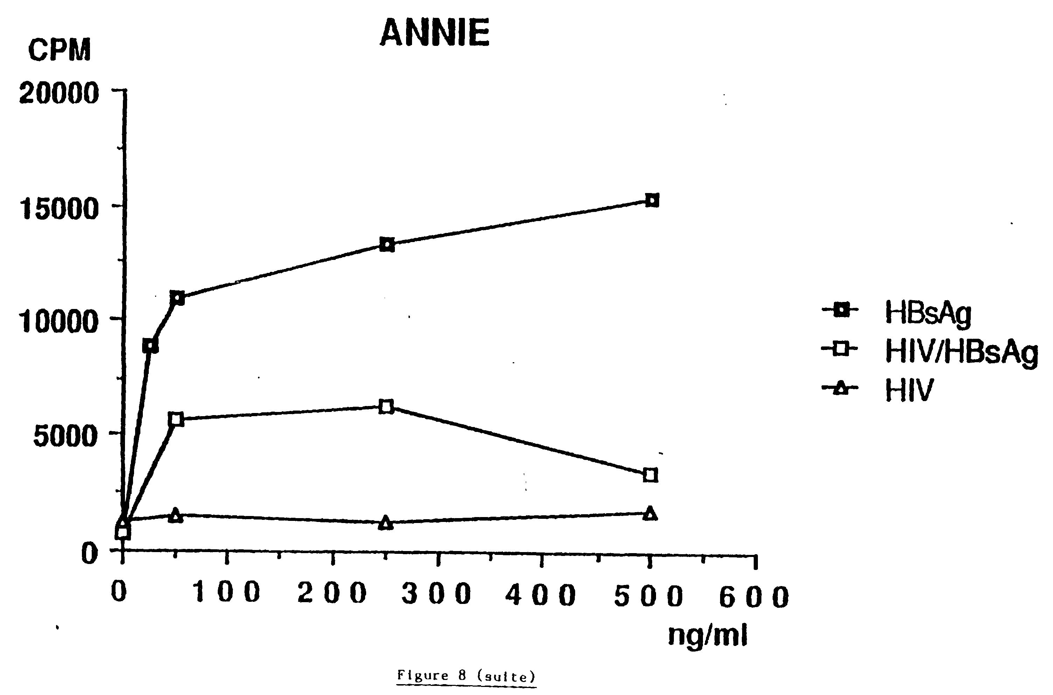

Les courbes de réponse caractéristiques des doses, obtenues après 21 jours après la première dose de vaccin, sont données à la figure 8. Les PBL de Mac H incorporent 8 fois plus de thymidine $^3$H en réponse à la stimulation avec une concentration optimale de particules HIV/HBsAg et 6 fois plus en réponse à la stimulation avec des particules HBsAg natives que des PBL non stimulés. Lorsqu'ils sont stiumulés avec la concentration optimale de HIV, les PBL de Mac H incorporent 5 fois plus de thymidine $^3$H que les PBL stimulés avec le milieu seul. Le singe Mac A de contrôle a été testé pour sa réponse proliférative en PBL 21 jours après la 3ème injection avec des particules natives HBsAg. Les PBL de Mac A prolifèrent en réponse à la stimulation soit avec les particules HBsAg natives, soit avec les particules HBsAg hybrides, mais ne prolifèrent pas en réponse à une stimulation avec HIV, quelle que soit la concentration utilisée.

La réponse proliférative des PBL à des immunozomes obtenus avec les glycoprotéines purifiées rgp160 ou gp160 natives a été testée. Les PBL de Mac H isolés 3 mois après la dernière injection sont stimulés avec HIV-1 purifié ou des immunozomes gp160 en utilisant des doses différentes (tableau 1). Au contraire, les gp160 recombinantes produites dans un système de vaccinia n'induisent pas de réponse proliférative. Les PBL de Mac A collectés 57 jours après la dernière injection ne prolifèrent pas lorsqu'ils sont stimulés avec l'un quelconque des antigènes. Ces résultats montrent que la maturation du fragment gp120 dans l'antigène particulier de l'invention est plus proche de la maturation de la glycoprotéine assemblée dans le virion ou dans les formes immunozomes que sous la forme monomérique.

La prolifération spécifique de PBL en réponse à HBsAg ou à HIV a également été suivie pour chaque animal pendant le protocole d'immunisation.

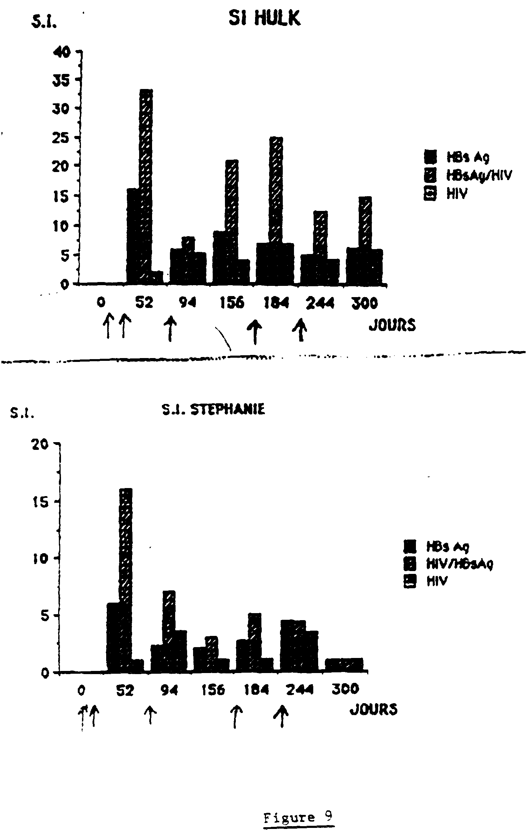

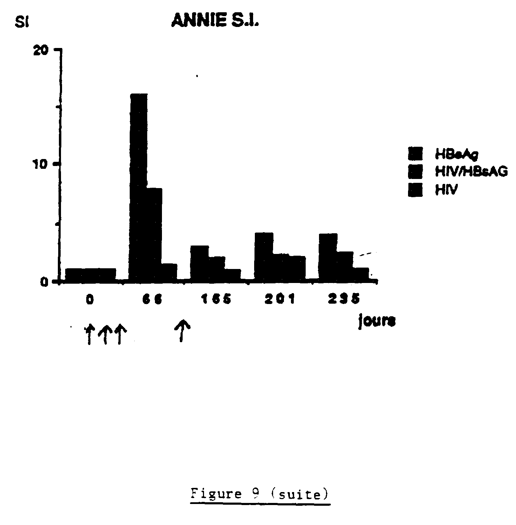

La figure 9 résume les résultats de la prolifération lymphocytaire en réponse à la stimulation avec HIV/HBsAg, avec HBsAg ou avec HIV. Plusieurs concentrations de chaque antigène ont été testées et les réponses ont été mesurées aux jours 4 et 5. Chez les macaques, le pic de prolifération est obtenu au jour 4 en utilisant HBsAg à une concentration optimale de 250 ng/ml et, aux jours 5 et 6 avec HIV en utilisant une concentration optimale de 500 ng/ml. La réponse proliférative maximale aux particules hybrides HIV/HBsAg et aux particules HBsAg est observée après la seconde injection et est toujours supérieure à la réponse obtenue après la stimulation avec des particules natives HBsAg pour les 2 macaques immunisés avec les particules hybrides.

La réponse de Mac H est meilleure vis-à-vis de HBsAg que la réponse de Mac S soit au niveau B (voir figure 7) soit au niveau T. La réponse proliférative suivant la troisième immunisation est plus faible qu'après la seconde injection. Cependant, un bon taux de cellules réagissant à l'antigène est retrouvé 3 mois après la troisième injection (Mac H). L'immunisation de rappel induit un léger accroissement de la réponse proliférative des PBL. Une différence importante est observée entre la réponse proliférative à HIV des PBL des deux macaques. Les PBL de Mac S prolifèrent de façon significative seulement après la troisième injection et après les doses de rappel alors que les PBL de Mac H prolifèrent de façon significative à la simulation HIV à un moment quelconque après l'immunisation.

Comme observé avec HBsAg, la réponse proliférative à HIV est faiblement accrue par le rappel mais persiste au même niveau 3 mois après la dernière injection (Mac H). En conséquence, le protocole d'immunisation peut être adapté en différant la dose de rappel.

La réponse des PBL de Mac S à HIV et à HBsAg est plus faible et est perdue 3 mois après la dernière injection probablement à cause de la faible quantité de cellules réagissant aux antigènes induite par la vaccination de ces animaux.

La comparaison de la réponse proliférative vis-à-vis de tous les antigènes testés montre que les PBL sont stimulés de la même façon par HIV ou par HBsAg native (Mac H). L'index de stimulation (SI) est comparable excepté au jour 52 où apparaît un nombre significativement plus élevé de cellules T spécifiques de HBsAg. Ces résultats démontrent que la réponse immune à HIV et à HBsAg médiée par les cellules, est induite chez les macaques après la vaccination avec des particules hybrides HIV/HBsAg et que la cinétique de cette réponse varie en fonction de l'animal.

En utilisant la particule HBs portant un fragment de la glycoprotéine d'enveloppe gp120 de HIV (AA384-472) comprenant le site de liaison CD4, des anticorps ont été obtenus ainsi qu'une réponse des cellules T, dirigée contre les deux parties de la protéine de fusion (HBsAg et HIV).

Les particules HBsAg constituent un bon véhicule pour la présentation des épitopes de HIV et permettent l'induction chez des macaques immunisés d'une réponse humorale ainsi que d'une réponse immune médiée par les cellules T, vis-à-vis d'un domaine fonctionnel de la protéine d'enveloppe gp120.

D'autres caractéristiques et propriétés de l'invention apparaîtront dans les figures qui suivent dont les légendes sont données ci-après.


- Figure 1


Vecteur d'expression et de clonage des fragments du gène de l'enveloppe de HIV. M13tg130 est un vecteur de clonage des fragments d'ADN de HIV. La région hachurée représente la partie N-terminale du gène de la β-galactosidase. Dans cette région, on trouve un adaptateur présentant de multiple sites de clonage. El : EcoRI, sm : SamI, Ss : SstI; EV : EcoRV, Sp : SphI, K : KpnI, Xb : XbaI, H : HindIII, B : BamHI, Sa : SalI, P : PstI.
PSVS, pM2S, pSV2S et pSVS XL sont des vecteurs d'expression.
Xh : XhoI


- Figure 2


Représentation schématique de la séquence d'acides aminés des fragments d'enveloppe de HIV fusionnée avec HBsAg. Le système de numérotation est celui qui a été donné par ALIZON et al dans l'article cité sous la référence (3). Les points et les flèches correspondent respectivement aux sites potentiels de glycosylation et aux cystéines conservées. Les séquences des 4 peptides représentent les régions conservées entre les isolats de HIV1 et de HIV2.


- Figure 3


Analyse Western-blot des particules recombinantes de HBsAg. Les particules recombinantes n°2 (A), n° 8 (b), n°6 (C), ont réagi avec : un sérum ant-p49a dilué à 1:500 (lignes A1, B1, C1), un antisérum anti-peptide 5 (ligne A2), un antisérum anti-peptide 6 (ligne B2), un antisérum anti-peptide 7 (ligne B3) à une dilution de 1:25 et à une dilution de 1:200 d'un anticorps monoclonal 18/7 (ligne C2). Les tailles sont données en kilodaltons.


- Figure 4


Test de neutralisation : la cinétique de la neutralisation <u>in vitro</u> des différents échantillons de virus (A, B, C) par un antisérum dilué à 1:50 obtenu après immunisation d'un lapin avec le recombinant n°8 (o---o) ou avec un sérum pré-immun (o—o). L'activité réverse transcriptase a été déterminée par l'incorporation de l$^3$HI thymidine triphosphate (en cpm). Sur le graphique D, chaque point représente le pourcentage d'inhibition RT pour A, B, C calculé comme suit

$$\frac{\text{cpm test RT sur culture avec sérum immun}}{\text{cpm test RT sur culture avec sérum pré-immun}} \times 100$$


- Figure 5


Neutralisation de HIV avec un sérum immun de lapin dirigé contre le recombinant n° 8. Des séra pré-immun (ligne continue) et immun (ligne pointillée) ont été testés pour connaître la neutralisation du virus dans des cultures cellulaires, en utilisant deux isolats différents LAV$_{BRU}$ (cercles) LAV$_{ELI}$ (triangles). L'activité reverse transcriptase a été mesurée après 7 à 10 jours dans des surnageants de cultures cellulaires.


- Figure 6


Sequences des protéines d'enveloppe des protéines d'enveloppe des rétrovirus SIV$_{mac}$, HIV-2, HIV-1. Dans cette figure les tirets apparaissant dans les séquences ont pour seul objet de permettre l'alignement des séquences. Ces tirets n'ont donc pas de signification scientifique.

Figure 7: <u>Titres en anticorps chez des macaques immunisés avec des particules hybrides HBsAg (S-H) ou des particules natives HBsAg (A).</u>


Mac S et H ont reçu une injection avec des particules purifiées HIV/HBsAg, selon une dose de 10μg ou 15μg respectivement aux jours 0,24 , 70, 167 et 203. Mac A a reçu une injection d'une dose de 15μg de particules HBsAg dérivées d'un plasma purifié aux jours 0,21, 43 et 165. La première dose a été donnée avec de l'adjuvant complet de Freund, les doses suivantes avec de l'adjuvant incomplet de Freund. Mac S et H ont reçu la quatrième dose sans adjuvant.

Les échantillons de sérum obtenus à différents moments après l'injection ont été dilués en série et testés pour la présence d'anticorps spécifiques de HBsAg ou de HIV en utilisant un test ELISA. Les titres sont exprimés par l'inverse de la dilution de serum qui donnait une valeur d'absorption 3 fois plus grande que la valeur obtenue avec le sérum preimmun. Chaque point est la moyenne de 4 déterminations.

Figure 8: Stimulation antigénique des lymphocytes

Les PBL ont été isolés par centrifugation avec Ficoll Hypaque à partir de sang héparinisé, de Mac H (immunisé 3 fois avec HIV/HBsAg) ou Mac A (immunise 3 fois avec HBsAg (contrôle)).

Les PBL ont été suspendus à 1,5 x $10^6$ cellules/ml dans un milieu RPMI 16-40 complété avec 10% de serum AB humain inactivé par la chaleur. 0,1ml ont été déposés en triple dans des puits à fond rond de plaques à 96 puits. Les PBL ont été stimulés avec 0,1ml de HIV-1 purifié sur un gradient de sucrose, avec des particules HBsAg purifiées sur 2 gradients successifs de chlorure de cesium. Chaque antigène a été testé à différentes concentrations. Quatre et cinq jours après la stimulation, chaque puits de PBL a été marqué avec de la thymidine $^3$H-T 1 μCi pendant 6 heures, les cellules ont été prélevées et la thymidine $^3$H incorporée a été déterminée par comptage de scintillation.

Figure 9: Suivi de la réponse proliférative des PBL des macaques.

Les lymphocytes ont été prélevés avant l'immunisation et après la seconde et la troisième immunisation, avant et après le rappel au jour indiqué sur la figure. Les flèches indiquent les jours d'immunisation des animaux. La capacité des lymphocytes d'incorporer de la thymidine $^3$H en réponse à un antigène exogène a été suivie comme sur la figure 8 et l'index de stimulation (S.I.) a été calculé en divisant le nombre de cpm de thymidine $^3$H incorporé par les cellules stimulées par le nombre de cpm incorporé par des cellules non stimulées. Dans ces tests le bruit de fond (cpm) était entre 100 et 2450 cpm. La réponse Blastogénique à la Con A donne à S.I. entre 5 et 129.

L'immunogénicité des particules préférées de l'invention peut également être attribuée à la glycosylation vraisemblable des peptides issus de HIV à l'occasion de l'expression des vecteurs recombinants sus-définis.

Dans ce qui précède la région contenant la séquence d'acides aminés immunogène de HIV et son site d'attachement à des récepteurs CD4 des lymphocytes T4 provenaient le plus souvent d'une séquence commune appartenant à une glycoprotéine de surface ou transmembranaire de HIV. Il va de soi que chacune de ces séquences peut provenir de régions éloignées l'une de l'autre dans les protéines naturelles de HIV. Dans ce dernier cas, elles consisteraient de préférence en une séquence recombinante ou en un polypeptide recombinant résultant de l'expression de deux séquences codées par les régions correspondantes du génome de HIV, voire même de HIV appartenant à des isolats différents.

Dans ce qui précède il a également été remarqué que de préférence on avait recours à des séquences issues de HIV mais bien conservées dans les divers isolats disponibles de HIV. Il va néamoins de soi que l'on peut avoir recours même à des séquences moins bien conservées d'un isolat à l'autre. Dans une telle hypothèse, l'invention concerne également des compositions de particules répondant à la définition qui en a été donnée plus haut, ces particules se distinguant cependant les unes des autres en ce qu'elles contiennent des séquences d'acides aminés communes avec des régions des glycoprotéines correspondantes d'isolats différents.

L'invention concerne enfin les séquences d'acides aminés préférées issues de HIV, qui étaient identifiées dans le cadre de la présente description, par conséquent aussi les séquences d'ADN qui codent pour elles (comme d'ailleurs les polypeptides produits par synthèse chimique et présentant des propriétés immunologiques semblables, ainsi que toutes séquences d'acides nucléiques codant pour ces polypeptides, qu'il s'agisse de séquences naturelles ou produites par synthèse nucléotidique).

En particulier les séquences d'acides aminés ci-dessus peuvent être utilisées pour la production de principes actifs de vaccins comprenant à titre de support une bactérie E.coli et, affleurant à la surface de cette bactérie, les séquences peptidiques sus-indiquées de HIV. Pour la production de tels vaccins on peut avoir recours à la technique décrite dans la demande de brevet européen déposée le 6 Mars 1987 et publiée sous le numéro 0242.243.

TABLEAU I

| Antisera de lapin dirigés contre les particules recombinantes HIV-HBsAg \ Antigène immobilisé | HBsAg dérivé du plasma | Pre-S2 Peptide (120-145) | Peptide 5 | Peptide 6 | Peptide 7 | Peptide F |
|---|---|---|---|---|---|---|
| n° 2 (2 x 3,5 µg)* | $10^{-4}$ | · 0 | $4.10^{-2}$ | - | - | - |
| n° 8 (2 x 3,5 µg)* | $10^{-3}$ | 0 | - | $2.10^{-3}$ | $5.10^{-2}$ | - |
| n° 6 (3 x 5 µg)* | $5.10^{-5}$ | $1,6.10^{-3}$ | - | - | - | $10^{-3}$ |

Tableau I : titres et spécificités des antiséra de lapin dirigés contre les particules recombinantes.
Les titres sont exprimés par la réciproque de la dilution du sérum donnant le triple de la densité obtenue avec un sérum pré-immun à la même dilution (=sérum témoin au jour 0).

\* : Les nombres entre parenthèse font référence à des doses d'injection.

EP 0 354 109 B1

| HIV [a] | | | IMMUNOZOMES [a] | | | GP160 [a] | | |
|---|---|---|---|---|---|---|---|---|
| Concentration | cpm [b] | S.I. [c] | Concentration | cpm [b] | S.I. [c] | Concentration | cpm [b] | S.I. [c] |
| ng/ml | 250 | - | ng/ml | 250 | - | ng/ml | 250 | - |
| 50 | 837 | 3.3 | 25 | 552 | 3.0 | 50 | 209 | 0.8 |
| 100 | 930 | 3.7 | 50 | 829 | 3.3 | 100 | 172 | 1.2 |
| 500 | 1512 | 6.0 | 250 | 1345 | 5.4 | 500 | 328 | 1.3 |
| 1000 | 1735 | 6.9 | 500 | 1545 | 6.1 | 1000 | 398 | 1.6 |

Tableau 2: réponses prolifératives des PBL de macaque H immunisé avec des particules de HIV/HBsAg après stimulation avec: HIV ou avec des glycoprotéines d'enveloppe de HIV.

a/ Les PBL ont été stimulés pendant 5 jours comme décrit à la figure 8 avec HIV purifié avec un gradient de sucrose, avec des immunozomes de gp160 purifiés à partir de cultures infectées, ou avec une gp160 purifiée recombinante produite dans un système de vaccinia-virus.
b/ cpm de $^3$H-thymidine incorporé après stimulation avec l'antigène

c/ S.I.

EP 0 354 109 B1

REFERENCES

1. Allan,J.S. et al.(1985) Science 228, 1091-1093.
2. Robey, W.G. et al.(1985) Science 228, 593-595.
3. Alizon,M. et al (1986) Cell 46, 63-74.
4. Starcich, B.R. et al (1986) Cell 45,637-648.
5. McDougal, J.S. et al (1986) Science 231, 382-395.
6. Robey, W.G. et al (1986) Proc.Natl. Acad. Sci. USA 83,7023-7027.
7. Lasky,L.A. et al (1986) Science 233, 209-212.
8. Putney, S.D. et al (1986) Science 234,1392-1395.
9. Chanh, T.C. et al (1986) EMBO J. 5,3065-3071.
10. Tiollais, P. et al (1985) Nature 317,489-495.
11. Michel, M.L. et al (1984)Proc.Natl.Acad.Sci. USA 81,7708-7712.
12. Neurath,A.R. et al (1984) Science 224,392-394.
13. Machida, A. et al (1984) Gastroenterology 86, 910-918.
14. Kieny,M.P. et al (1983) Gene 26, 91-99.
15. Urlaub, G. et al (1980) Proc. Natl. Acad. Sci.USA 77, 4216-4220.
16. Parker, B.A. et al (1979)J.Virol.31,360-369.
17. Subramani,S.(1981) Molecular and Cellular Biology 1, 854-864.
18. Michel, M.L. et al (1985) Biotechnology 3, 561-566.
19. Gerin, J.L. et al (1983)Proc.Natl.Acad.Sci.USA 80,2365-2369.
20. Heermann, K.H. et al (1984)J. Virol. 52,396-402.
21. Charbit, A. et al (1987)J.Immunology 139, 1658-1664.
22. Montagnier, L. et al (1984) In Human T-Cell Leukemia/Lymphoma Virus, ed. Gallo, R.C., Essex, M. & Gross, L. (Cold Spring Harbor Laboratory, Cold Spring Harbor, New-York),pp. 363-370.
23. Rey, M.A. et al (1984) Biochemical and Biophysical Research Communications 121, 126-133.
24. Maddon, P.J. et al (1986) Cell 47, 333-348.
25. Lasky, L.A. et al (1987) Cell 50, 975-985.
26. Gallaher, W.R. et al (1987)Cell 50, 327-328.
27. Milich, D.R. et al (1986)J.Exp. Med. 164,532-547.
28. Valenzuela, P. et al(1985) Biotechnology 3, 323-326.
29. Delpeyroux, F. et al(1986) Science 233, 472-475.
30. Cease, K.B.et al (1987) Proc.Natl.Acad.Sci.USA 84, 4249-4253.
31. Krohn, K.D et al (1987) Proc.Natl.Acad.Sci.USA 84, 4994-4998.
32. Kowalski, M. et al (1987)Science 237, 1351-1355.

**Revendications**

1.  Vecteur recombinant, comprenant un fragment d'acide nucléique correspondant à la région S et le cas échéant à tout ou partie de la région pré-S de l'antigène majeur de surface du virus de l'hépatite B, et permettant, lorsqu'il est introduit dans un hôte cellulaire eucaryote compétent, et à l'occasion de la culture de l'hôte cellulaire ainsi transformé, d'induire la production de particules ayant les caractéristiques morphologiques essentielles de l'antigène HBsAg, et, de préférence l'excrétion de ces particules dans le milieu de culture, caractérisé

    -   en ce qu'il comprend en outre une séquence nucléotidique exogène codant pour une séquence d'acides aminés immunogène induisant des anticorps neutralisants vis-à-vis d'un rétrovirus de la famille de HIV ou susceptible d'être reconnue par de tels anticorps, comprenant au moins un épitope susceptible d'être reconnu par les lymphocytes B, et au moins un épitope susceptible d'être reconnu par les lymphocytes T, et
    -   en ce que ladite séquence nucléotidique exogène est soit insérée dans ladite région pré-S, soit substituée à tout ou partie de la dite région pré-S.

2.  Vecteur recombinant selon la revendication 1, caractérisé en ce que la séquence nucléotidique exogène code pour une séquence d'acides aminés immunogène comprenant en outre au moins un site d'attachement au récepteur CD4.

3.  Vecteur recombinant selon l'une quelconque des revendications 1 ou 2, caractérisé en ce que le susdit fragment d'acide nucléique est placé sous le contrôle d'un promoteur reconnu par les polymérases d'un hôte cellulaire eucaryote.

4. Vecteur recombinant selon la revendication 3, caractérisé en ce que le promoteur est un promoteur issu de SV40.

5. Vecteur recombinant selon l'une quelconque des revendications 1 à 4, caractérisé en ce que la séquence nucléotidique insérée est substituée à la totalité de la région pré-S.

6. Vecteur recombinant selon l'une quelconque des 'revendications 1 à 4, caractérisé en ce que la séquence nucléotidique exogène est insérée dans la partie pré-S2 de la région pré-S.

7. Vecteur recombinant selon la revendication 6, caractérisé en ce que la région pré-S2 de part et d'autre de la séquence insérée a pour l'essentiel été délétée.

8. Vecteur recombinant selon l'une quelconque des revendications 1 à 7, caractérisé en ce que la séquence nucléotidique exogène insérée comprend une séquence codant pour l'une des séquences d'acides aminés suivantes :

$$X_1 \quad \text{CHIRQIINTWHKVGKNVYLPPREGDLTC} \quad Z_1$$
$$X_2 \quad \text{CHIKQIINTWHKVGRNVYLPPREGELSC} \quad Z_2$$
$$X_3 \quad \text{CRIKQFINMWQEVGKAMYAPPISGQIRC} \quad Z_3$$

dans lesquelles les couples $X_1$, $Z_1$ ; $X_2$, $Z_2$ et $X_3$, $Z_3$ représentent soit des groupements COOH ou $NH_2$ soit des séquences d'acides aminés normalement respectivement associées aux parties centrales correspondantes des séquences sus-indiquées dans les glycoprotéines des rétrovirus SIV, HIV-2, HIV-2 qui normalement les contiennent.

9. Vecteur recombinant selon l'une quelconque des revendications 1 à 8, caractérisé en ce que la séquence nucléotidique exogène insérée code pour tout ou partie de l'une des séquences suivantes :

RGEFLYCKMNWFLNWVEDRSLTTQKPKERHKRNYVPCHIRQIINTWHKVGKNVYLP

PREGDLTCNSTVTSLIANINWTDG

RGEFLYCNMTWFLNWIENKTHRNYAPCHIKQIINTWHKVGRNVYLPPREGELSCNS

TVTSIIANIDWQNN

GGEFFYCNSTQLFNSTWFNSTWSTEGSNNTEGSDTITLPCRIKQFINMWQEVGKAM

YAPPISGQIRCSSNITGLLLTRDGGNN

10. Vecteur recombinant selon la revendication 6, Caractérisé en ce qu'il est dérivé du vecteur pM2S que contient la séquence pré-S1.

11. Vecteur recombinant selon la revendication 7, caractérisé en ce qu'il est dérivé du vecteur pSV2S que ne contient pas la séquence pré-S1.

12. Particules recombinantes présentant les caractéristiques morphologiques essentielles des particules de HBsAg, et comprenant, affleurant à la surface de ces particules, l'essentiel de la séquence d'acides aminés (S) correspondant à la région S et le cas échéant à tout ou partie de la séquence d'acides aminés correspondant à la région pré-S du génome du virus de l'hépatite B, en amont de ladite séquence d'acides aminés (S), ces particules recombinantes étant caractérisées en ce qu'elles comprennent en outre, une séquence d'acides aminés immunogène induisant des anticorps neutralisants vis-à-vis d'un rétrovirus de la famille HIV ou suceptible d'être reconnue par de tels anticorps, cette séquence comprenant elle-même au moins un épitope susceptible d'être reconnu par les lymphocytes B, ou au moins un épitope susceptible d'être reconnu par les lymphocytes T, cette séquence immunogène étant, soit insérée dans la séquence d'acides aminés pré-S, soit substituée tout ou partie de la protéine pré-S.

13. Particules recombinantes selon la revendication 12, caractérisées en ce que la séquence d'acides aminés immunogène comprend en outre un site d'attachement au récepteur CD4 de lymphocytes T4 humains.

**14.** Particules recombinantes selon l'une quelconque des revendications 12 ou 13, caractérisées en ce que la séquence d'acides aminés immunogène est soit insérée dans la séquence d'acides aminés codée par la région pré-S2 du génome du virus de l'hépatite B, soit substituée à tout ou partie de la séquence d'acides aminés pré-S2.

**15.** Particules recombinantes selon l'une quelconque des revendication 12 ou 13, caractérisées en ce que la séquence d'acides aminés pré-S2 est, pour l'essentiel, délétée.

**16.** Particules recombinantes selon l'une quelconque des revendications 12 à 15, caractérisées par une activité immunogène lui permettant d'induire in vivo la production d'anticorps protecteurs contre un rétrovirus de la classe des rétrovirus HIV pathogènes pour l'homme.

**17.** Particules recombinantes selon l'une quelconque des revendications 12 à 16, caractérisées en ce qu'elles ont pour l'essentiel perdu la faculté d'induire in vivo la production d'anticorps neutralisants à l'égard du virus de l'hépatite B.

**18.** Particules recombinantes selon l'une quelconque des revendications 12 à 17, caractérisées en ce que la séquence d'acides aminés immunogène insérée est choisie parmi les séquences ci-dessous :

$$X_1 \; CHIRQIINTWHKVGKNVYLPPREGDLTC \; Z_1$$
$$X_2 \; CHIKQIINTWHKVGRNVYLPPREGELSC \; Z_2$$
$$X_3 \; CRIKQFINMWQEVGKAMYAPPISGQIRC \; Z_3$$

dans lesquelles les couples $X_1$, $Z_1$ ; $X_2$, $Z_2$ et $X_3$, $Z_3$ représentent soit des groupements COOH ou $NH_2$ soit des séquences d'acides aminés normalement respectivement associées aux parties centrales correspondantes des séquences sus-indiquées dans les glycoprotéines des rétrovirus SIV, HIV-2, HIV-1 qui nornalement les contiennent.

**19.** Particules recombinantes selon l'une quelconque des revendications 12 à 18, caractérisées en ce que la séquence d'acides aminés immunogène insérée est choisie parmis les séquences ci-dessous :

```
RGEFLYCKMNWFLNWVEDRSLTTQKPKERHKRNYVPCHIRQIINTWHKVGKNVYLP

PREGDLTCNSTVTSLIANINWTDG

RGEFLYCNMTWFLNWIENKTHRNYAPCHIKQIINTWHKVGRNVYLPPREGELSCNS

TVTSIIANIDWQNN

GGEFFYCNSTQLFNSTWFNSTWSTEGSNNTEGSDTITLPCRIKQFINMWQEVGKAM

YAPPISGQIRCSSNITGLLLTRDGGNN
```

**20.** Hôte cellulaire d'origine eucaryote, caractérisé en ce qu'il est transformé par un vecteur recombinant selon l'une quelconque des revendications 1 à 11, le rendant apte, lorsqu'il est mis en culture, à excréter des particules selon l'une quelconque des revendications 12 à 19.

**21.** Hôte cellulaire selon la revendication 20, caractérisé en ce qu'il s'agit d'une cellule de mammifère.

**22.** Hôte cellulaire selon la revendication 20, caractérisé en ce qu'il s'agit d'une levure.

**23.** Fragment immunogène d'acides aminés, caractérisé en ce qu'il comprend tout ou partie de l'un des enchaînements ci-dessous :

```
RGEFLYCKMNWFLNWVEDRSLTTQKPKERHKRNYVPCHIRQIINTWHKVGKNVYLP

PREGDLTCNSTVTSLIANINWTDG

RGEFLYCNMTWFLNWIENKTHRNYAPCHIKQIINTWHKVGRNVYLPPREGELSCNS

TVTSIIANIDWQNN

GGEFFYCNSTQLFNSTWFNSTWSTEGSNNTEGSDTITLPCRIKQFINMWQEVGKAM

YAPPISGQIRCSSNITGLLLTRDGGNN
```

**24.** Acide nucléique, caractérisé en ce qu'il code pour l'une des séquences d'acides aminés immunogènes selon la revendication 23.

**25.** Composition vaccinante destinée à la protection contre une infection par un rétrovirus de la famille de HIV, caractérisée en ce que son principe actif est formé, des particules recombinantes selon l'une quelconque des revendications 12 à 19, en association avec un véhicule pharmaceutique acceptable.

**26.** Procédé de préparation d'un vecteur recombinant, caractérisé par les étapes suivantes :

- la recombinaison in vitro et le cas échéant sous le contrôle d'un promoteur reconnu par les polymérases d'un hôte cellulaire eucaryote, d'une part d'une séquence nucléotidique exogène codant pour une séquence d'acides aminés immunogéne, induisant des anticorps neutralisants vis-à-vis d'un rétrovirus de la famille de HIV ou suceptible d'être reconnue par de tels anticorps, et comprenant au moins un épitope susceptible d'être reconnu par les lymphocytes B, au moins un épitope susceptible d'être reconnu par les lymphocytes T et d'autre part, un enchaînement de nucléotides correspondant à la région S et le cas échéant à tout ou partie de la région pré-S, du génome du virus de l'hépatite B.
- la récupération du vecteur recombinant formé comprenant les éléments, tels qu'ils ont été définis plus haut.

**27.** Procédé selon la revendication 26 , caractérisé en ce que la séquence d'acides aminés immunogène correspond à l'insérat défini dans l'une quelconque des revendications 1 à 11.

**28.** Composition pour le diagnostic d'une infection due au rétrovirus HIV par la détection de la présence d'anticorps neutralisants dans un milieu biologique prélevé chez un patient, comprenant des particules recombinantes selon l'une quelconque des revendications 18 ou 19 et/ou un fragment immunogène selon la revendication 23 ou un mélange de ces composants.

**29.** Test pour le diagnostic in vitro d'une infection due à HIV par la détection de la présence d'anticorps neutralisants dans un milieu biologique prélevé chez un patient, caractérisé par ;

- la mise en contact, dans des conditions appropriées, d'une composition selon la revendication 28 avec le milieu biologique testé,
- la détection de la formation d'un complexe antigène-anticorps.

**30.** Procédé pour la production de particules recombinantes selon l'une quelconque des revendications 12 à 19 caractérisé par :

- la transformation d'un hôte cellulaire déterminé avec un vecteur recombinant selon l'une quelconque des revendications 1 à 11,
- la culture de l'hôte cellulaire transformé sur un ni lieu approprié et
- la récupération des particules recombinantes formées.

**31.** Utilisation des fragment immunogènes d'acides aminés selon la revendication 23, seuls ou en mélange, en tant qu'antigènes d'immuno-essai pour la détection in vitro d'anticorps, en particulier d'anticorps neutralisants, notamment en suivi de vaccination.

**32.** Méthode de dosage in vitro de la présence d'anticorps dirigés contre un rétrovirus HIV dans un milieu biologique, en particulier pour le dosage d'anticorps neutralisants en suivi de vaccination, comprenant les étapes suivantes :

- la mise en contact des fragments immunogènes d'acides aminés selon revendication 23, marqués de façon à permettre le dosage ultérieur, avec le milieu biologique à tester, dans des conditions appropriées pour permettre la réaction,
- le dosage desdits fragments ayant réagi avec le milieu.

33. Trousse (Kit) pour le diagnostic in vitro d'anticorps anti-HIV dans un milieu biologique et en particulier d'anticorps neutralisants en suivi de vaccination, caractérisée en ce qu'elle comprend

- des fragments immunogènes d'acides aminés selon revendication 23,
- des moyens adaptés pour la révélation de la réaction antigène-anticorps.

## Patentansprüche

1. Rekombinanter Vektor enthaltend ein Nucleinsäurefragment, das der S-Region und gegebenenfalls der gesamten oder einem Teil der prä-S-Region des Hauptoberflächenantigens des Hepatitis B-Virus entspricht und bei Einführung in eine kompetente eukaryontische Wirtszelle und Kultivierung der so transformierten Wirtszelle die Induktion der Produktion von Partikeln, die die wesentlichen morphologischen Eigenschaften des Antigens HBsAg aufweisen, erlaubt und vorzugsweise die Ausscheidung dieser Partikel in das Kulturmedium, dadurch gekennzeichnet,

- daß er weiterhin eine exogene Nucleotidsequenz enthält, die eine immunogene Aminosäuresequenz codiert, die neutralisierende Antikörper induziert gegen ein Retrovirus der HIV-Familie oder eines, das durch derartige Antikörper erkannt werden kann, enthaltend mindestens ein Epitop, das von den B-Lymphocyten erkannt werden kann, und mindestens ein Epitop, das von den T-Lymphocyten erkannt werden kann, und
- daß die exogene Nucleotidsequenz entweder in der prä-S-Region inseriert ist oder gegen die gesamte oder einen Teil der prä-S-Region ausgetauscht ist.

2. Rekombinanter Vektor nach Anspruch 1, dadurch gekennzeichnet, daß die exogene Nucleotidsequenz eine immunogene Aminosäuresequenz codiert, die weiterhin mindestens eine Stelle zur Anheftung an den CD4-Rezeptor enthält.

3. Rekombinanter Vektor nach einem der Ansprüche 1 oder 2, dadurch gekennzeichnet, daß das Nucleinsäurefragment unter die Kontrolle eines Promotors gestellt ist, der von den Polymerasen einer eukaryontischen Wirtszelle erkannt wird.

4. Rekombinanter Vektor nach Anspruch 3, dadurch gekennzeichnet, daß der Promotor von SV40 stammt.

5. Rekombinanter Vektor nach einem der Ansprüche 1 bis 4, dadurch gekennzeichnet, daß die insertierte Nucleotidsequenz gegen die gesamte prä-S-Region ausgetauscht ist.

6. Rekombinanter Vektor nach einem der Ansprüche 1 bis 4, dadurch gekennzeichnet, daß die exogene Nucleotidsequenz in dem prä-S2-Bereich der prä-S-Region insertiert ist.

7. Rekombinanter Vektor nach Anspruch 6, dadurch gekennzeichnet, daß die prä-S2-Region beiderseits der insertierten Sequenz im wesentlichen deletiert ist.

8. Rekombinanter Vektor nach einem der Ansprüche 1 bis 7, dadurch gekennzeichnet, daß die insertierte exogene Nucleotidsequenz eine Sequenz enthält, die eine der folgenden Aminosäuresequenzen codiert:

$$X_1 \ \text{CHIRQIINTWHKVGRNVYLPPREGDLTC} \ Z_1$$
$$X_2 \ \text{CHIKQIINTWHKVGRNVYLPPREGELSC} \ Z_2$$
$$X_3 \ \text{CRIKQFINMWQEVGKAMYAPPISGQIRC} \ Z_3$$

in denen die Paare $X_1$, $Z_1$; $X_2$, $Z_2$ und $X_3$, $Z_3$ entweder COOH- oder $NH_2$-Gruppen darstellen oder Aminosäuresequenzen, die normalerweise jeweils assoziiert sind mit den mittleren Bereichen, die den oben genannten Sequenzen entsprechen, in Glycoproteinen der Retroviren SIV, HIV-2 oder HIV-1, die diese normalerweise enthalten.

9. Rekombinanter Vektor nach einem der Ansprüche 1 bis 8, dadurch gekennzeichnet, daß die insertierte exogene Nucleotidsequenz die gesamte oder einen Teil einer der folgenden Sequenzen codiert:

```
RGEFLYCKMNWFLNWVEDRSLTTQKPKERHKRNYVPCHIRQIIHTWHKVGKNVYLP

PREGDLTCNSTVTSLIANINWTDG

RGEFLYCNMTWFLNWIENKTHRNYAPCHIKQIINTWHKVGRNVYLPPREGELSCNS

TVTSIIANIDWQHN

GGEFFYCNSTQLFNSTWFNSTWSTEGSNNTEGSDTITLPCRIKQFINMWQEVGKAM

YAPPISGQIRCSSNITGLLLTRDGGNN
```

10. Rekombinanter Vektor nach Anspruch 6, dadurch gekennzeichnet, daß er von dem Vektor pM2S abgeleitet ist, der die prä-S1-Sequenz enthält.

11. Rekombinanter Vektor nach Anspruch 7, dadurch gekennzeichnet, daß er von dem Vektor pSV2S abgeleitet ist, der nicht die prä-S1-Sequenz enthält.

12. Rekombinante Partikel, die die wesentlichen morphologischen Eigenschaften von HBsAg-Partikeln aufweisen und die, an der Oberfläche dieser Partikel auftretend, den Hauptbestandteil der Aminosäuresequenz (S) enthalten, die der S-Region oder gegebenenfalls der gesamten oder einem Teil der Aminosäuresequenz, die die prä-S-Region des Genoms des Hepatitis B-Virus darstellt, entspricht, stromaufwärts der Aminosäuresequenz (S), wobei diese Partikel dadurch gekennzeichnet sind, daß sie weiterhin eine immunogene Aminosäuresequenz umfassen, die neutralisierende Antikörper induziert gegen ein Retrovirus der Familie HIV oder gegen eines, das durch derartige Antikörper erkannt werden kann, wobei diese Sequenz ihrerseits mindestens ein Epitop enthält, das von den B-Lymphocyten erkannt werden kann, oder mindestens ein Epitop, das von den T-Lymphocyten erkannt werden kann, und diese immunogene Sequenz entweder in der prä-S-Aminosäuresequenz insertiert ist oder gegen das gesamte oder einen Teil des prä-S-Proteins ausgetauscht ist.

13. Rekombinante Partikel nach Anspruch 12, dadurch gekennzeichnet, daß die immunogene Aminosäuresequenz weiterhin eine Stelle zur Anheftung an den CD4-Rezeptor von menschlichen T4-Lymphozyten enthält.

14. Rekombinante Partikel nach einem der Ansprüche 12 oder 13, dadurch gekennzeichnet, daß die immunogene Aminosäuresequenz entweder in der Aminosäuresequenz insertiert ist, die durch die prä-S2-Region des Genoms des Hepatitis B-Virus codiert wird, oder gegen die gesamte oder einen Teil der prä-S2-Aminosäuresequenz ausgetauscht ist.

15. Rekombinante Partikel nach einem der Ansprüche 12 oder 13, dadurch gekennzeichnet, daß die prä-S2-Aminosäuresequenz im wesentlichen deletiert ist.

16. Rekombinante Partikel nach einem der Ansprüche 12 bis 15, gekennzeichnet durch eine immunogene Aktivität, die ihnen erlaubt, in vivo die Bildung protektiver Antikörper gegen ein Retrovirus der für Menschen pathogenen Klasse der HIV-Retroviren zu induzieren.

17. Rekombinante Partikel nach einem der Ansprüche 12 bis 16, dadurch gekennzeichnet, daß sie im wesentlichen die Fähigkeit verloren haben, in vivo die Bildung neutralisierender Antikörper hinsichtlich des Hepatitis B-Virus zu induzieren.

18. Rekombinante Partikel nach einem der Ansprüche 12 bis 17, dadurch gekennzeichnet, daß die insertierte immunogene Aminosäuresequenz ausgewählt ist aus den nachstehenden Sequenzen:

$$X_1 \quad CHIRQIINTWHKVGKNVYLPPREGDLTC \quad Z_1$$
$$X_2 \quad CHIKQIINTWHKVGRNVYLPPREGELSC \quad Z_2$$
$$X_3 \quad CRIKQFINMWQEVGKAMYAPPISGQIRC \quad Z_3$$

in denen die Paare $X_1$, $Z_1$; $X_2$, $Z_2$ und $X_3$, $Z_3$ entweder COOH- oder $NH_2$-Gruppen oder Aminosäuresequenzen darstellen, die normalerweise jeweils mit den mittleren Bereichen assoziiert sind, die den oben angegebenen Sequenzen entsprechen, in Glycoproteinen der Retroviren SIV, HIV-2 oder HIV-1, die diese normalerweise enthalten.

19. Rekombinante Partikel nach einem der Ansprüche 12 bis 18, dadurch gekennzeichnet, daß die inserierte immunogene Aminosäuresequenz aus den nachstehenden Sequenzen ausgewählt ist:

```
RGEFLYCKMNWFLNWVEDRSLTTQKPKERHKRNYVPCHIRQIINTWHKVGKNVYLP
PREGDLTCNSTVTSLIANINWTDG
RGEFLYCNHTWFLNWIENKTHRNYAPCHIKQIINTWHKVGRNVYLPPREGELSCNS
TVTSIIANIDWQNN
GGEFFYCNSTQLFNSTWFNSTWSTEGSNNTEGSDTITLPCRIKQFINMWQEVGKAM
YAPPISGQIRCSSHITGLLLTRDGGNN
```

20. Wirtszelle eukaryontischen Ursprungs, dadurch gekennzeichnet, daß sie mit einem rekombinanten Vektor nach einem der Ansprüche 1 bis 11 transformiert ist, wodurch sie zur Ausscheidung von Partikeln nach einem der Ansprüche 12 bis 19 geeignet ist, wenn sie kultiviert wird.

21. Wirtszelle nach Anspruch 20, dadurch gekennzeichnet, daß es sich um eine Säugerzelle handelt.

22. Wirtszelle nach Anspruch 20, dadurch gekennzeichnet, daß es sich um eine Hefezelle handelt.

23. Immunogenes Aminosäurefragment, dadurch gekennzeichnet, daß es die gesamte oder einen Teil der nachfolgenden Abfolgen enthält:

```
RGEFLYCKMNWFLNWVEDRSLTTQKPKERHKRNYVPCHIRQIINTWHKVGKNVYLP
PREGDLTCNSTVTSLIANINWTDG
RGEFLYCNHTWFLNWIENKTHRNYAPCHIKQIINTWHKVGRNVYLPPREGELSCNS
TVTSIIANIDWQNN
GGEFFYCNSTQLFNSTWFNSTWSTEGSNNTEGSDTITLPCRIKQFINMWQEVGKAM
YAPPISGQIRCSSHITGLLLTRDGGNN
```

24. Nucleinsäure, dadurch gekennzeichnet, daß sie eine der immunogenen Aminosäuresequenzen nach Anspruch 23 codiert.

25. Impfstoffzusammensetzung für den Schutz gegen Infektionen durch ein Retrovirus der HIV-Familie, dadurch gekennzeichnet, daß ihr Wirkstoff aus rekombinanten Partikeln nach einem der Ansprüche 12 bis 19 besteht in Verbindung mit einem pharmazeutisch verträglichen Träger.

26. Verfahren zur Herstellung eines rekombinanten Vektors, gekennzeichnet durch die folgenden Schritte:

-   in vitro-Rekombination und gegebenenfalls unter der Kontrolle eines Promotors, der von den Polymerasen einer eukaryontischen Wirtszelle erkannt wird, eines Teils einer exogenen Nucleotidsequenz, codierend eine immunogene Aminosäuresequenz, die neutralisierende Antikörper induziert gegen ein Retrovirus der HIV-Familie oder gegen eines, das von derartigen Antikörpern erkannt werden kann, und die mindestens ein Epitop enthält, das von den B-Lymphocyten erkannt werden kann, oder mindestens ein Epitop, das von den T-Lymphocyten erkannt werden kann, und andererseits einer Abfolge von Nucleotiden, die der S-Region entspricht und gegebenenfalls dem gesamten oder einem Teil der prä-S-Region des Genoms des Hepatitis B-Virus,

- Wiedergewinnung des gebildeten rekombinanten Vektors, der die weiter oben definierten Elemente enthält.

27. Verfahren nach Anspruch 26, dadurch gekennzeichnet, daß die immunogene Aminosäuresequenz der in einem der Ansprüche 1 bis 11 definierten Insertion entspricht.

28. Zusammensetzung für die Diagnose einer durch einen HIV-Retrovirus hervorgerufenen Infektion mittels des Nachweises der Anwesenheit neutralisierender Antikörper in einem biologischen Medium, das einem Patienten entnommen wurde, wobei die Zusammensetzung rekombinante Partikel nach einem der Ansprüche 18 oder 19 und/oder ein immunogenes Fragment nach Anspruch 23 enthält oder ein Gemisch dieser Komponenten.

29. Testverfahren zur in vitro-Diagnose einer durch HIV hervorgerufenen Infektion mittels des Nachweises der Anwesenheit neutralisierender Antikörper in einem biologischen Medium, das einem Patienten entnommen wurde, gekennzeichnet durch:

- das Inkontaktbringen einer Zusammensetzung nach Anspruch 28 mit dem zu untersuchenden biologischen Medium unter geeigneten Bedingungen,
- den Nachweis der Bildung eines Antigen-Antikörper-Komplexes.

30. Verfahren zur Herstellung rekombinanter Partikel nach einem der Ansprüche 12 bis 19, gekennzeichnet durch:

- die Transformation einer ausgewählten Wirtszelle mit einem rekombinanten Vektor nach einem der Ansprüche 1 bis 11,
- Kultivierung der transformierten Wirtszelle in einem geeigneten Medium und
- Gewinnung der gebildeten rekombinanten Partikel.

31. Verwendung von immunogenen Aminosäurefragmenten nach Anspruch 23, einzeln oder als Gemisch, als Antigen in einem Immuntest für den in vitro-Nachweis von Antikörpern, insbesondere von neutralisierenden Antikörpern, besonders im Anschluß an eine Impfung.

32. Verfahren zur in vitro-Mengenbestimmung der Anwesenheit von Antikörpern, die gegen ein HIV-Retrovirus gerichtet sind, in einem biologischen Medium, insbesondere für die Mengenbestimmung von neutralisierenden Antikörpern nach einer Impfung, umfassend folgende Schritte:

- Inkontaktbringen von immunogenen Aminosäurefragmenten nach Anspruch 23, die derart markiert sind, daß sie die spätere Mengenbestimmung erlauben, mit dem zu untersuchenden biologischen Medium unter geeigneten Bedingungen, die die Reaktion erlauben,
- Mengenbestimmung derjenigen Fragmente, die mit dem Medium reagiert haben.

33. Testbesteck zur in vitro-Diagnose von anti-HIV-Antikörpern in einem biologischen Medium und insbesondere von neutralisierenden Antikörpern nach einer Impfung, dadurch gekennzeichnet, daß es

- immunogene Aminosäurefragmente nach Anspruch 23, und
- Mittel, die für den Nachweis der Antigen-Antikörper-Reaktion angepaßt sind, enthält.

## Claims

1. Recombinant vector comprising a nucleic acid fragment corresponding to region 5 and, if appropriate, to all or part of the pre-S region of the major surface antigen of the virus of hepatitis B, and which permits, when it is introduced into a suitable eucaryotic cell host, and upon culture of the cell host thus transformed, production to be induced of particles having the essential morphological characteristics of the antigen HBsAg and, preferably, excretion of these particles in the culture medium, characterised in that :

- it comprises in addition an exogenic nucleotide sequence coding for a sequence of immunogenic amino acids inducing neutralising antibodies in relation to a retrovirus of the HIV family, or capable of being recognised by such antibodies, comprising at least one epitope capable of being recognised by the B lymphocytes, and at least one epitope capable of being recognised by the T lymphocytes, and
- in that the said exogenic nucleotide sequence is either inserted into the said pre-S region, or substituted for all or part of the said pre-S region.

2. Recombinant vector according to claim 1, characterised in that the exogenic nucleotide sequence codes for an immunogenic sequence of amino acids comprising in addition at least one site for attachment to the receptor CD4.

3. Recombinant vector according to any one of claims 1 or 2, characterised in that the abovementioned fragment of nucleic acid is placed under the control of a promoter recognised by the polymerases of a eucaryotic cell host.

4. Recombinant vector according to claim 3, characterised in that the promoter is a promoter originating from SV40.

5. Recombinant vector according to any one of claims 1 to 4, characterised in that the inserted nucleotide sequence is substituted for all of the pre-S region.

6. Recombinant vector according to any one of claims 1 to 4, characterised in that the exogenic nucleotide sequence is inserted in the pre-S2 portion of the pre-S region.

7. Recombinant vector according to claim 6, characterised in that the pre-S2 region on either side of the inserted sequence has been substantially deleted.

8. Recombinant vector according to any one of claims 1 to 7, characterised in that the inserted exogenic nucleotide sequence comprises a sequence coding for one of the following amino acid sequences:

$$X_1 \; \text{CHIRQIINTWHKVGKNVYLPPREGDLTC} \; Z_1$$

$$X_2 \; \text{CHIKQIINTWHKVGRNVYLPPREGELSC} \; Z_2$$

$$X_3 \; \text{CRIKQFINMWQEVGKAMYAPPISGQIRC} \; Z_3,$$

in which the couples $X_1$, $Z_1$, $X_2$, $Z_2$ and $X_3$, $Z_3$ represent either COOH or $NH_2$ groupings, or amino acid sequences normally respectively associated with the corresponding central portions of the sequences indicated above, in the glycoproteins of the retroviruses SIV, HIV - 2, HIV - 2 which normally contain them.

9. Recombinant vector according to any one of claims 1 to 8, characterised in that the inserted exogenic nucleotide sequence codes for all or part of one of the following sequences:

RCEFLYCKMNWFLNWVEDRSLTTQKPKERHKRNYVPCHIRQIINTWHKVCKNVYLPPREG

DLTCNSTVTSLIANINWTDG

RCEFLYCNMTWFLNWIENKTHRNYAPCHIKQIINTWHKVCRNVYLPPREGELSCNSTVTS

IIANIDWQNN

GGEFFYCNSTQLFNSTWFNSTWSTEGSNNTEGSDTITLPCRIKQFINMWQEVGKAMYAPP

ISGQIRCSSNITGLLLTRDGGNN

10. Recombinant vector according to claim 6, characterised in that it is derived from the vector pM2S, which contains the pre-S1 sequence.

11. Recombinant vector according to claim 7, characterised in that it is derived from the vector pSV2S, which does not contain the pre-S1 sequence.

12. Recombinant particles having the essential morphological characteristics of the HBsAg particles, and comprising, fitting flush on the surface of these particles, the substantial proportion of the sequence of amino acids (S) corresponding to the S region and, if appropriate, corresponding to all or part of the sequence of amino acids corresponding to the pre-S region of the genome of the virus of hepatitis B, upstream of the said sequence of amino acids (S), these recombinant particles being characterised in that they comprise in addition a sequence of immunogenic amino acids inducing neutralising antibodies against a retrovirus of the HIV family or capable of being recognised by such antibodies, this sequence itself comprising at least one epitope capable of being recognised by the B lymphocytes, or at least one epitope capable of being recognised by the T lymphocytes, this immunogenic sequence being either inserted into the sequence of pre-S amino acids or substituted for all or part of the pre-S protein.

13. Recombinant particles according to claim 12, characterised in that the immunogenic sequence of amino acids comprises in addition a site for attachment to the CD4 receptor of human T4 lymphocytes.

14. Recombinant particles according to any one of claims 12 or 13, characterised in that the immunogenic sequence of amino acids is either inserted into the sequence of amino acids coded by the pre-S2 region of the genome of the hepatitis B virus, or substituted for all or part of the pre-S2 sequence of amino acids.

15. Recombinant particles according to any one of claims 12 or 13, characterised in that the pre-S2 sequence of amino acids is substantially deleted.

16. Recombinant particles according to any one of claims 12 to 15, characterised by an immunogenic activity enabling it to induce, in vivo, the production of protective antibodies against a retrovirus of the class of HIV retroviruses pathogenic to man.

17. Recombinant particles according to any one of claims 12 to 16, characterised in that they have substantially lost the faculty of inducing in vivo the production of neutralising antibodies against the hepatitis B virus.

18. Recombinant particles according to any one of claims 12 to 17, characterised in that the immunogenic sequence of amino acids inserted is selected from the sequences below:

$$X_1 \ \text{CHIRQIINTWHKVGKNVYLPPREGDLTC} \ Z_1$$

$$X_2 \ \text{CHIKQIINTWHKVGRNVYLPPREGELSC} \ Z_2$$

$$X_3 \ \text{CRIKQFINMWQEVGKAMYAPPISGQIRC} \ Z_3,$$

in which the couples $X_1$, $Z_1$, $X_2$, $Z_2$ and $X_3$, $Z_3$ represent either COOH or $NH_2$ groupings) or amino acid sequences normally respectively associated with the corresponding central portions of the sequences indicated above, in the glycoproteins of the retroviruses SIV, HIV - 2, HIV - 2 which normally contain them.

19. Recombinant particles according to any one of claims 12 to 18, characterised in that the immunogenic sequence of amino acids inserted is selected from the following sequences:

RCEFLYCKMNWFLNWVEDRSLTTQKPKERHKRNYVPCHIRQIINTWHKVCKNVYLPPREG

DLTCNSTVTSLIANINWTDG

RCEFLYCNMTWFLNWIENKTHRNYAPCHIKQIINTWHKVCRNVYLPPREGELSCNSTVTS

IIANIDWQNN

GGEFFYCNSTQLFNSTWFNSTWSTEGSNNTEGSDTITLPCRIKQFINMWQEVGKAMYAPP

ISGQIRCSSNITGLLLTRDGGNN.

20. Cell host of eucaryotic origin, characterised in that it is transformed by a recombinant vector according to any one of claims 1 to 11, rendering it capable, when it is cultured, of excreting particles according to any one of claims 12 to 19.

21. Cell host according to claim 20, characterised in that a mammalian cell is involved.

22. Cell host according to claim 20, characterised in that a yeast is involved.

23. Immunogenic fragment of amino acids, characterised in that it comprises all or part of one of the following chains:

RCEFLYCKMNWFLNWVEDRSLTTQKPKERHKRNYVPCHIRQIINTWHKVCKNVYLPPREG

DLTCNSTVTSLIANINWTDG

RCEFLYCNMTWFLNWIENKTHRNYAPCHIKQIINTWHKVCRNVYLPPREGELSCNSTVTS

IIANIDWQNN

GGEFFYCNSTQLFNSTWFNSTWSTEGSNNTEGSDTITLPCRIKQFINMWQEVGKAMYAPP

ISGQIRCSSNITGLLLTRDGGNN.

24. Nucleic acid, characterised in that it codes for one of the immunogenic sequences of amino acids according to claim 23.

25. Vaccination compound intended for protection against an infection by a retrovirus of the HIV family, characterised in that its active ingredient is formed from the recombinant particles according to any one of claims 12 to 19, in association with an acceptable pharmaceutical carrier.

26. Method of preparation of a recombinant vector, characterised by the following stages:

- recombination in vitro and, if appropriate, under the control of a promoter recognised by the polymerases of a eucaryotic cell host, on the one hand of an exogenic nucleotide sequence coding for an immunogenic sequence of amino acids, inducing neutralising antibodies against a retrovirus of the HIV virus, or capable of being recognised by such antibodies, and comprising at least one epitope capable of being recognised by the B lymphocytes, at least one epitope capable of being recognised by T lymphocytes and on the other hand, a nucleotide sequence corresponding to the S region and, if appropriate, to all or part of the pre-S region of the genome of the hepatitis B virus,

- recovery of the recombinant vector formed comprising the elements as defined above.

27. Method according to claim 26, characterised in that the immunogenic sequence of amino acids corresponds to the insert defined in any one of claims 1 to 11.

28. Composition for diagnosis of an infection due to the HIV retrovirus by means of detecting the presence of neutralising antibodies in a biological medium taken from a patient, comprising recombinant particles according to any one of claims 18 or 19 and/or an immunogenic fragment according to claim 23, or a mixture of these ingredients.

29. Test for in-vitro diagnosis of an infection due to HIV, by means of detecting the presence of neutralising antibodies in a biological medium taken from a patient, characterised by:

- bringing a composition according to claim 28 into contact, under appropriate conditions, with the biological medium tested;
- detection of the formation of an antigen/antibody complex.

30. Method for manufacturing recombinant particles according to any one of claims 12 to 19, characterised by:

- transformation of a specific cell host with a recombinant vector according to any one of claims 1 to 11;
- culture of the transformed cell host on a suitable medium, and
- recovery of the recombinant particles formed.

31. Utilisation of the immunogenic fragments of amino acids according to claim 23, alone or in mixture, as immuno-test antigens for the in-vitro detection of antibodies, in particular neutralising antibodies, particularly following vaccination.

32. Method of in-vitro dosage of the presence of antibodies directed against an HIV retrovirus in a biological medium, in particular for dosage of neutralising antibodies following vaccination, comprising the following stages:

- bringing immunogenic fragments of amino acids according to claim 23 into contact, marked to as to allow subsequent dosage with the biological medium to be tested, in appropriate conditions to enable reaction;
- dosage of said fragments which have reacted with the medium.

33. Kit for in-vitro diagnosis of anti-HIV antibodies in a biological medium, and in particular of neutralising antibodies following vaccination, characterised in that it comprises

- immunogenic fragments of amino acids according to claim 23;
- means adapted to reveal the antigen/antibody reaction.

Figure 1

Figure 2

Figure 3

Figure 4

Figure 5

EP 0 354 109 B1

SIV.MAC  TVTSLIANIMNVTGCHGVSITMSAEVAELYRL---ELGDYKLVEITPIGLAPTKVKEVTTGGTSKHKGVFVLG--FLGFLATAGSAMGAAS---LTVTAQSR  550
HIV.2    TVTSIIANIDWONNHGTHTTFSAEVAELYRL---ELGDYKLVEITPIGFAPTKEKRYSSAHG-RHTRGVFVLG--FLGFLATAGSAMGAAS---LTVSAQSR  541
HIV.1    HTTGLLLTRDGGNNNTCSEIFRPGGGDMRDNWRSELYKYKVVKIEPLGVAPTKAKRR----VVQREKRAVGI-GALFLGFLGAAGSTMGARSMTLTVQA--R  547

clone B ←           EGP               ↑           ↑ TMP

SIV.MAC  TLLAGIVQQQQQLLDVVKRQQELLRLTVWGTKNLQTRVSAIEKYLKDQAQLNAWGCAFRQVCHTTVPW-----PNASLTPDWNNETWQEWERKVDFLEANIT  655
HIV.2    TLLAGIVQQQQQLLDVVKRQQELLRLTVWGTKNLCARVTAIEKYLQDQARLNSWGCAFRQVCHTTVPW-----VNDSLAPDWDNHTWQEWEKQVRYLEANIS  630
HIV.1    QLLSGIVQQQNNLLRAICAQQHLLQLTVWGIKQLQARILAVERYLKDQQLLGIWGCSGKLICTTAVPWNASWSNKSLEQIWNHTTWMEWDREINNYTSLIH  640

SIV.MAC  ALLEEAQIQQEKNMYELQKLNSWDVFGNWFDLTSWIKYIQYGIYIIVGVILLRIVIYIVQMLARLRQGYRPV-FSSPPSYFQWTHTQQQPALPTKEGKKGD  755
HIV.2    KSLEQAQIQQEKNMYELQKLNSWDIFGNWFDLTSWVKYIQYGVLIIVAVIALRIVIYVVQMLSRLRKGYRPV-FSSPFGVIQQIHIHKDRGQPANEETEED  730
HIV.1    SLIEESQNQQEKNEQELLELDKWASLWNWFNITNWLWYIKIFIMIVGGLVGLRIVFAVLSIVNRVRQGYSPLSFCT--------HLPTPRGPDRPEGIEEE  741

SIV.MAC  GGGSCGNSSWNQIEYIHFLIRQLIPLLTIWLFSNCRTLLSRAYQILQPIFQRLSATLRAIREVLRLELTYLQYGWSYFQEAVQAAQRSATETLAGAWGEL  853
HIV.2    GGSNGGQRVWPWPIAYIHFLIRQLIRLLT-RLVSIQRQLLSRSFLTLQLIYQNLRDW-------LRLRTAFLQYGCEWIQEAFQAAARATRETLAGAGRGL  831
HIV.1    GGERDRDRSIRLVNGSLA-LIWDDLRSLCLFSYHRLRDLLLIVTRIVELLG--RRGWEA-----LKVWNNLLQYWSQELKNSAVSLLNATAIAVAEGTDRV  834

SIV.MAC  WEALQRGGRWILAIPRRIRQGLELILL  802
HIV.2    WRVLERIGRGILAVPRRIRQGAEIALL  850
HIV.1    IEVVQGAGRAIRHIPRRIRQGLERILL  861

Figure 6 (suite n°1)

35

Figure 7

EP 0 354 109 B1

HULK

Figure 8

ng/ml

**ANNIE**

Figure 8 (suite)

Figure 9

Figure 9 (suite)